# EUROPÄISCHE PATENTSCHRIFT

(11) **EP 3 271 340 B1**
(45) Veröffentlichungstag und Bekanntmachung des Hinweises auf die Patenterteilung: **02.10.2019**
(21) Anmeldenummer: 16714246.2
(22) Anmeldetag: 14.03.2016
(51) Int. Cl.: C07D 271/113, A01N 43/26

(54) **SALZE VON N-(1,3,4-OXADIAZOL-2-YL) ARYLCARBONSÄUREAMIDEN UND IHRE VERWENDUNG ALS HERBIZIDE**
SALTS OF N-(1,3,4-OXADIAZOL-2-YL) ARYL CARBOXYLIC ACID AMIDES AND USE OF SAME AS HERBICIDES
SELS DE N-(1,3,4-OXADIAZOLE-2-YL) AMIDES D'ACIDES CARBONIQUE D'ARYLE ET LEURS UTILISATIONS EN TANT QU'HERBICIDE

(30) Priorität: 17.03.2015 EP 15159483
(43) Veröffentlichungstag der Anmeldung: 24.01.2018
(73) Patentinhaber: Bayer CropScience Aktiengesellschaft, 40789 Monheim am Rhein (DE)
(72) Erfinder: KÖHN, Arnim, 55270 Klein-Winternheim (DE); BRAUN, Ralf, 76857 Ramberg (DE); AHRENS, Hartmut, 63329 Egelsbach (DE); WALDRAFF, Christian, 61118 Bad Vilbel (DE); HEINEMANN, Ines, 65719 Hofheim (DE); DIETRICH, Hansjörg, 65835 Liederbach am Taunus (DE); GATZWEILER, Elmar, 61231 Bad Nauheim (DE); ROSINGER, Christopher Hugh, 65719 Hofheim (DE)
(74) Vertreter: BIP Patents
(86) Internationale Anmeldenummer: PCT/EP2016/055396
(87) Internationale Veröffentlichungsnummer: WO 2016/146561

(56) Entgegenhaltungen:
- WO-A1-2012/126932
- WO-A1-2013/124245
- WO-A1-2013/164331
- US-A- 4 416 683
- Anonym: "Daten zu Vergleichsversuchen Nr. 13719550.9 - 1462", European Register Office , 9. Juli 2015 (2015-07-09), Seiten 1-5, XP002757333, Gefunden im Internet: URL:https://register.epo.org/application?d ocumentId=EXQ5465S2290FI4&number=EP1371955 0&lng=en&npl=true [gefunden am 2016-05-11]

## Beschreibung

Die Erfindung betrifft das technische Gebiet der Herbizide, insbesondere das der Herbizide zur selektiven Bekämpfung von Unkräutern und Ungräsern in Nutzpflanzenkulturen.

WO 2012/126932 A1 beschreibt N-(1,3,4-Oxadiazol-2-yl)benzamide und ihre Verwendung als Herbizide. Die dort beschriebenen Wirkstoffe zeigen nicht immer eine ausreichende Wirkung gegen Schadpflanzen und/oder sie sind zum Teil nicht ausreichend verträglich mit einigen wichtigen Kulturpflanzen, wie Getreidearten, Mais und Reis. Aufgabe der vorliegenden Erfindung ist es daher, weitere herbizid wirksame Wirkstoffe bereitzustellen. Diese Aufgabe wird durch die nachfolgend beschriebenen erfindungsgemäßen Salze von N-(1,3,4-Oxadiazol-2-yl)arylcarbonsäureamiden gelöst.

Beschrieben werden Salze von N-(1,3,4-Oxadiazol-2-yl)benzamiden der Formel (I) 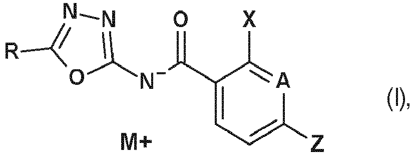 worin
A bedeutet N oder CY,
R bedeutet Wasserstoff, (C₁-C₆)-Alkyl, R¹O-(C₁-C₆)-Alkyl, CH₂R⁶, (C₃-C₇)-Cycloalkyl, Halogen-(C₁-C₆)-alkyl, (C₂-C₆)-Alkenyl, Halogen-(C₂-C₆)-alkenyl, (C₂-C₆)-Alkinyl, Halogen-(C₂-C₆)-alkinyl, OR¹, NHR¹, Methoxycarbonyl, Ethoxycarbonyl, Methoxycarbonylmethyl, Ethoxycarbonylmethyl, Methylcarbonyl, Trifluormethylcarbonyl, Dimethylamino, Acetylamino, Methylsulfenyl, Methylsulfinyl, Methylsulfonyl oder jeweils durch s Reste aus der Gruppe Halogen, Nitro, Cyano, (C₁-C₆)-Alkyl, Halogen-(C₁-C₆)-alkyl, (C₃-C₆)-Cycloalkyl, S(O)ₙ-(C₁-C₆)-Alkyl, (C₁-C₆)-Alkoxy, Halogen-(C₁-C₆)-alkoxy, (C₁-C₆)-Alkoxy-(C₁-C₄)-alkyl substituiertes Heteroaryl, Heterocyclyl, Benzyl oder Phenyl,
X bedeutet Nitro, Halogen, Cyano, Formyl, Rhodano, (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₃-C₆)-alkinyl, (C₃-C₆)-Cycloalkyl, Halogen-(C₃-C₆)-cycloalkyl, (C₃-C₆)-Cycloalkyl-(C₁-C₆)-alkyl, Halogen-(C₃-C₆)-cycloalkyl-(C₁-C₆)-alkyl, COR¹, COOR¹, OCOOR¹, NR¹COOR¹, C(O)N(R¹)₂, NR¹C(O)N(R¹)₂, OC(O)N(R¹)₂, C(O)NR¹OR¹, OR¹, OCOR¹, OSO₂R², S(O)ₙR², SO₂OR¹, SO₂N(R¹)₂, NR¹SO₂R², NR¹COR¹, (C₁-C₆)-Alkyl-S(O)ₙR², (C₁-C₆)-Alkyl-OR¹, (C₁-C₆)-Alkyl-OCOR¹, (C₁-C₆)-Alkyl-OSO₂R², (C₁-C₆)-Alkyl-CO₂R¹, (C₁-C₆)-Alkyl-SO₂OR¹, (C₁-C₆)-Alkyl-CON(R¹)₂, (C₁-C₆)-Alkyl-SO₂N(R¹)₂, (C₁-C₆)-Alkyl-NR¹COR¹, (C₁-C₆)-Alkyl-NR¹SO₂R², NR₁R₂, P(O)(OR⁵)₂, CH₂P(O)(OR⁵)₂, (C₁-C₆)-Alkyl-Heteroaryl, (C₁-C₆)-Alkyl-Heterocyclyl, wobei die beiden letzt genannten Reste jeweils durch s Reste Halogen, (C₁-C₆)-Alkyl, Halogen-(C₁-C₆)-alkyl, S(O)ₙ-(C₁-C₆)-Alkyl, (C₁-C₆)-Alkoxy, Halogen-(C₁-C₆)-alkoxy, und wobei Heterocyclyl n Oxogruppen trägt,
Y bedeutet Wasserstoff, Nitro, Halogen, Cyano, Rhodano, (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₂-C₆)-alkinyl, (C₃-C₆)-Cycloalkyl, (C₃-C₆)-Cycloalkenyl, Halogen-(C₃-C₆)-cycloalkyl, (C₃-C₆)-Cycloalkyl-(C₁-C₆)-alkyl, Halogen-(C₃-C₆)-cycloalkyl-(C₁-C₆)-alkyl, COR¹, COOR¹, OCOOR¹, NR¹COOR¹, C(O)N(R¹)₂, NR¹C(O)N(R¹)₂, OC(O)N(R¹)₂, CO(NOR¹)R¹, NR¹SO₂R², NR¹COR¹, OR¹, OSO₂R², S(O)ₙR², SO₂OR¹, SO₂N(R¹)₂ (C₁-C₆)-Alkyl-S(O)ₙR², (C₁-C₆)-Alkyl-OR¹, (C₁-C₆)-Alkyl-OCOR¹, (C₁-C₆)-Alkyl-OSO2R², (C₁-C₆)-Alkyl-CO₂R¹, (C₁-C₆)-Alkyl-CN, (C₁-C₆)-Alkyl-SO₂OR¹, (C₁-C₆)-Alkyl-CON(R¹)₂, (C₁-C₆)-Alkyl-SO₂N(R¹)₂, (C₁-C₆)-Alkyl-NR¹COR¹, (C₁-C₆)-Alkyl-NR¹SO₂R², N(R¹)₂, P(O)(OR⁵)₂, CH₂P(O)(OR⁵)₂, (C₁-C₆)-Alkyl-Phenyl, (C₁-C₆)-Alkyl-Heteroaryl, (C₁-C₆)-Alkyl-Heterocyclyl, Phenyl, Heteroaryl oder Heterocyclyl, wobei die letzten 6 Reste jeweils durch s Reste aus der Gruppe Halogen, Nitro, Cyano, (C₁-C₆)-Alkyl, Halogen-(C₁-C₆)-alkyl, (C₃-C₆)-Cycloalkyl, S(O)ₙ-(C₁-C₆)-Alkyl, (C₁-C₆)-Alkoxy, Halogen-(C₁-C₆)-alkoxy, (C₁-C₆)-Alkoxy-(C₁-C₄)-alkyl und Cyanomethyl substituiert sind, und wobei Heterocyclyl n Oxogruppen trägt,
Z bedeutet Halogen, Cyano, Rhodano, (C₁-C₆)-Alkyl, (C₁-C₆)-Alkoxy, Halogen-(C₁-C₆)-alkyl, (C₂-C₆)-Alkenyl, Halogen-(C₂-C₆)-alkenyl, (C₂-C₆)-Alkinyl, Halogen-(C₂-C₆)-alkinyl, (C₃-C₆)-Cycloalkyl, Halogen-(C₃-C₆)-cycloalkyl, (C₃-C₆)-Cycloalkyl-(C₁-C₆)-alkyl, Halogen-(C₃-C₆)-cycloalkyl-(C₁-C₆)-alkyl, COR¹, COOR¹, OCOOR¹, NR¹COOR¹, C(O)N(R¹)₂, NR¹C(O)N(R¹)₂, OC(O)N(R¹)₂, C(O)NR¹OR¹, OSO₂R², S(O)ₙR², SO₂OR¹, SO₂N(R¹)₂, NR¹SO₂R², NR¹COR¹, (C₁-C₆)-Alkyl-S(O)ₙR², (C₁-C₆)-Alkyl-OR¹, (C₁-C₆)-Alkyl-OCOR¹, (C₁-C₆)-Alkyl-OSO₂R², (C₁-C₆)-Alkyl-CO₂R¹, (C₁-C₆)-Alkyl-SO₂OR¹, (C₁-C₆)-Alkyl-CON(R¹)₂, (C₁-C₆)-Alkyl-SO₂N(R¹)₂, (C₁-C₆)-Alkyl-NR¹COR¹, (C₁-C₆)-Alkyl-NR¹SO₂R², N(R¹)₂, P(O)(OR⁵)₂, Heteroaryl, Heterocyclyl oder Phenyl, wobei die letzten drei Reste jeweils durch s Reste aus der Gruppe Halogen, Nitro, Cyano, (C₁-C₆)-Alkyl, Halogen-(C₁-C₆)-alkyl, (C₃-C₆)-Cycloalkyl, S(O)ₙ-(C₁-C₆)-Alkyl, (C₁-C₆)-Alkoxy oder Halogen-(C₁-C₆)-alkoxy substituiert sind, und wobei Heterocyclyl n Oxogruppen trägt, oder
   Z kann auch Wasserstoff bedeuten, falls Y für den Rest S(O)ₙR² steht,
R¹ bedeutet Wasserstoff, (C₁-C₆)-Alkyl, (C₁-C₆)-Halogenalkyl, (C₂-C₆)-Alkenyl, (C₂-C₆)-Halogenalkenyl, (C₂-C₆)-Alkinyl, (C₂-C₆)-Halogenalkinyl, (C₃-C₆)-Cycloalkyl, (C₃-C₆)-Cycloalkenyl, (C₃-C₆)-Halogencycloalkyl, (C₁-C₆)-Alkyl-O-(C₁-C₆)-alkyl, (C₃-C₆)-Cycloalkyl-(C₁-C₆)-alkyl, Phenyl, Phenyl-(C₁-C₆)-alkyl, Heteroaryl, (C₁-C₆)-Alkyl-Heteroaryl, Heterocycl, (C₁-C₆)-Alkyl-Heterocyclyl, (C₁-C₆)-Alkyl-O-Heteroaryl, (C₁-C₆)-Alkyl-O-Heterocyclyl, (C₁-C₆)-Alkyl-NR³-Heteroaryl, (C₁-C₆)-Alkyl-NR³-Heterocyclyl wobei die 21 letztgenannten Reste durch s Reste aus der Gruppe bestehend aus Cyano, Halogen, Nitro, Rhodano, OR³, S(O)ₙR⁴, N(R³)₂, NR³OR³, COR³, OCOR³, SCOR⁴, NR³COR³, NR³SO₂R⁴, CO₂R³, COSR⁴, CON(R³)₂ und (C₁-C₄)-Alkoxy-(C₂-C₆)-alkoxycarbonyl substituiert sind, und wobei Heterocyclyl n Oxogruppen trägt,
R² bedeutet (C₁-C₆)-Alkyl, (C₁-C₆)-Halogenalkyl, (C₂-C₆)-Alkenyl, (C₂-C₆)-Halogenalkenyl, (C₂-C₆)-Alkinyl, (C₂-C₆)-Halogenalkinyl, (C₃-C₆)-Cycloalkyl, (C₃-C₆)-Cycloalkenyl, (C₃-C₆)-Halogencycloalkyl, (C₁-C₆)-Alkyl-O-(C₁-C₆)-alkyl, (C₃-C₆)-Cycloalkyl-(C₁-C₆)-alkyl, Phenyl, Phenyl-(C₁-C₆)-alkyl, Heteroaryl, (C₁-C₆)-Alkyl-Heteroaryl, Heterocyclyl, (C₁-C₆)-Alkyl-Heterocyclyl, (C₁-C₆)-Alkyl-O-Heteroaryl, (C₁-C₆)-Alkyl-O-Heterocyclyl, (C₁-C₆)-Alkyl-NR³-Heteroaryl, (C₁-C₆)-Alkyl-NR³-Heterocyclyl wobei die 21 letztgenannten Reste durch s Reste aus der Gruppe bestehend aus Cyano, Halogen, Nitro, Rhodano, OR³, S(O)ₙR⁴, N(R³)₂, NR³OR³, COR³, OCOR³, SCOR⁴, NR³COR³, NR³SO₂R⁴, CO₂R³, COSR⁴, CON(R³)₂ und (C₁-C₄)-Alkoxy-(C₂-C₆)-alkoxycarbonyl substituiert sind, und wobei Heterocyclyl n Oxogruppen trägt,
R³ bedeutet Wasserstoff, (C₁-C₆)-Alkyl, (C₂-C₆)-Alkenyl, (C₂-C₆)-Alkinyl, (C₃-C₆)-Cycloalkyl oder (C₃-C₆)-Cycloalkyl-(C₁-C₆)-alkyl,
R⁴ bedeutet (C₁-C₆)-Alkyl, (C₂-C₆)-Alkenyl oder (C₂-C₆)-Alkinyl,
R⁵ bedeutet Methyl oder Ethyl,
R⁶ bedeutet Acetoxy, Acetamido, N-Methylacetamido, Benzoyloxy, Benzamido, N-Methylbenzamido, Methoxycarbonyl, Ethoxycarbonyl, Benzoyl, Methylcarbonyl, Piperidinylcarbonyl, Morpholinylcarbonyl, Trifluormethylcarbonyl, Aminocarbonyl, Methylaminocarbonyl, Dimethylaminocarbonyl, (C₁-C₆)-Alkoxy, (C₃-C₆)-Cycloalkyl oder jeweils durch s Reste aus der Gruppe Methyl, Ethyl, Methoxy, Trifluormethyl und Halogen substituiertes Heteroaryl, Heterocyclyl oder Phenyl,
n bedeutet 0, 1 oder 2,
s bedeutet 0, 1, 2 oder 3,
M⁺ bedeutet ein Kation ausgewählt aus der Gruppe bestehend aus Natrium-Ion, Kalium-Ion, Lithium-Ion, Magnesium-Ion, Calcium-Ion, NH₄⁺-Ion, (2-Hydroxyeth-1-yl)ammonium-lon, Bis-N,N-(2-hydroxyeth-1-yl)-ammonium-Ion, Tris-N,N,N-(2-hydroxyeth-1-yl)ammonium-lon, Tetra-N,N,N,N-(2-hydroxyeth-1-yl)ammonium-Ion, N-(2-Hydroxyeth-1-yl)-tris-N,N,N-methylammonium-Ion, Methylammonium-Ion, Dimethylammonium-Ion, Trimethylammonium-Ion, Tetramethylammonium-Ion, Ethylammonium-Ion, Diethylammonium-Ion, Triethylammonium-Ion, Tetraethylammonium-Ion, Isopropylammonium-Ion, Diisopropylammonium-Ion, Tetrapropylammonium-Ion, Tetrabutyl-ammonium-Ion, Tetraoctylammonium-Ion; 2-(2-Hydroxyeth-1-oxy)eth-1-yl-ammonium-Ion, Di-(2-hydroxy-eth-1-yl)-ammonium-Ion, Trimethylbenzylammonium-Ion, Triethylbenzylammonium-Ion, Tri-((C₁-C₄)-alkyl)-sulfonium-Ion, Benzylammonium-Ion, 1-Phenylethylammonium-Ion, 2-Phenylethylammonium-Ion, Diisopropylethylammonium-Ion, Pyridinium-Ion, Piperidinium-Ion, Imidazolium-Ion, Morpholinium-Ion, 1,8-Diazabicyclo-[5.4.0]undec-7-enium-Ion.

In der Formel (I) und allen nachfolgenden Formeln können Alkylreste mit mehr als zwei Kohlenstoffatomen geradkettig oder verzweigt sein. Alkylreste bedeuten z.B. Methyl, Ethyl, n- oder i-Propyl, n-, i-, t- oder 2-Butyl, Pentyle, Hexyle, wie n-Hexyl, i-Hexyl und 1,3-Dimethylbutyl. Analog bedeutet Alkenyl z.B. Allyl, 1-Methylprop-2-en-1-yl, 2-Methyl-prop-2-en-1-yl, But-2-en-1-yl, But-3-en-1-yl, 1-Methyl-but-3-en-1-yl und 1-Methyl-but-2-en-1-yl. Alkinyl bedeutet z.B. Propargyl, But-2-in-1-yl, But-3-in-1-yl, 1-Methyl-but-3-in-1-yl. Die Mehrfachbindung kann sich jeweils in beliebiger Position des ungesättigten Rests befinden. Cycloalkyl bedeutet ein carbocyclisches, gesättigtes Ringsystem mit drei bis sechs C-Atomen, z.B. Cyclopropyl, Cyclobutyl, Cyclopentyl oder Cyclohexyl. Analog bedeutet Cycloalkenyl eine monocyclische Alkenylgruppe mit drei bis sechs Kohlenstoffringgliedern, z.B. Cyclopropenyl, Cyclobutenyl, Cyclopentenyl und Cyclohexenyl, wobei sich die Doppelbindung an beliebiger Position befinden kann.

Halogen steht für Fluor, Chlor, Brom oder Iod.

Heterocyclyl bedeutet einen gesättigten, teilgesättigten oder vollständig ungesättigten cyclischen Rest, der 3 bis 6 Ringatome enthält, von denen 1 bis 4 aus der Gruppe Sauerstoff, Stickstoff und Schwefel stammen, und der zusätzlich durch einen Benzoring annelliert sein kann. Beispielsweise steht Heterocyclyl für Piperidinyl, Pyrrolidinyl, Tetrahydrofuranyl, Dihydrofuranyl und Oxetanyl,
Heteroaryl bedeutet einen aromatischen cyclischen Rest, der 3 bis 6 Ringatome enthält, von denen 1 bis 4 aus der Gruppe Sauerstoff, Stickstoff und Schwefel stammen, und der zusätzlich durch einen Benzoring annelliert sein kann. Beispielsweise steht Heteroaryl für Benzimidazol-2-yl, Furanyl, Imidazolyl, Isoxazolyl, Isothiazolyl, Oxazolyl, Pyrazinyl, Pyrimidinyl, Pyridazinyl, Pyridinyl, Benzisoxazolyl, Thiazolyl, Pyrrolyl, Pyrazolyl, Thiophenyl, 1,2,3-Oxadiazolyl, 1,2,4-Oxadiazolyl, 1,2,5-Oxadiazolyl, 1,3,4-Oxadiazolyl, 1,2,4-Triazolyl, 1,2,3-Triazolyl, 1,2,5-Triazolyl, 1,3,4-Triazolyl, 1,2,4-Triazolyl, 1,2,4-Thiadiazolyl, 1,3,4-Thiadiazolyl, 1,2,3-Thiadiazolyl, 1,2,5-Thiadiazolyl, 2H-1,2,3,4-Tetrazolyl, 1H-1,2,3,4-Tetrazolyl, 1,2,3,4-Oxatriazolyl, 1,2,3,5-Oxatriazolyl, 1,2,3,4-Thiatriazolyl und 1,2,3,5-Thiatriazolyl.

Ist eine Gruppe mehrfach durch Reste substituiert, so ist darunter zu verstehen, daß diese Gruppe durch ein oder mehrere gleiche oder verschiedene der genannten Reste substituiert ist. Analoges gilt für den Aufbau von Ringsystemen durch verschiedene Atome und Elemente.

Die Definition des Kations M⁺ ist so zu verstehen, dass die Salze der Formel (I) in ladungsneutraler Form vorliegen. Dies bedeutet im Falle einwertiger Kationen, dass ein Anion als Gegenanion vorliegt. Im Falle mehrwertiger Kationen, z.B zwei- oder dreiwertiger Kationen, liegen zwei oder drei Anionen als Gegenanionen vor.

Beschrieben werden bevorzugte Verbindungen der Formel (I), worin
A bedeutet N oder CY,
R bedeutet Wasserstoff, (C₁-C₆)-Alkyl, (C₃-C₇)-Cycloalkyl, Halogen-(C₁-C₆)-alkyl, (C₃-C₇)-Cycloalkylmethyl, Methoxycarbonylmethyl, Ethoxycarbonylmethyl, Acetylmethyl, Methoxymethyl, Methoxyethyl, Benzyl, Pyrazin.2-yl, Furan-2-yl, Tetrahydrofuran-2-yl, Morpholin, Dimethylamino oder durch s Reste aus der Gruppe Methyl, Methoxy, Trifluormethyl und Halogen substituiertes Phenyl;
X bedeutet Nitro, Halogen, Cyano, (C₁-C₆)-Alkyl, Halogen-(C₁-C₆)-alkyl, (C₃-C₆)-Cycloalkyl, OR¹, S(O)ₙR², (C₁-C₆)-Alkyl-S(O)ₙR², (C₁-C₆)-Alkyl-OR¹, (C₁-C₆)-Alkyl-CON(R¹)₂, (C₁-C₆)-Alkyl-SO₂N(R¹)₂, (C₁-C₆)-Alkyl-NR¹COR¹, (C₁-C₆)-Alkyl-NR¹SO₂R², (C₁-C₆)-Alkyl-Heteroaryl, (C₁-C₆)-Alkyl-Heterocyclyl, wobei die beiden letzt genannten Reste jeweils durch s Reste Halogen, (C₁-C₆)-Alkyl, Halogen-(C₁-C₆)-alkyl, S(O)ₙ-(C₁-C₆)-Alkyl, (C₁-C₆)-Alkoxy, Halogen-(C₁-C₆)-alkoxy substituiert sind, und wobei Heterocyclyl n Oxogruppen trägt,
Y Wasserstoff, Nitro, Halogen, Cyano, (C₁-C₆)-Alkyl, (C₁-C₆)-Halogenalkyl, OR¹, S(O)ₙR², SO₂N(R¹)₂, N(R¹)₂, NR¹SO₂R², NR¹COR¹, (C₁-C₆)-Alkyl-S(O)ₙR², (C₁-C₆)-Alkyl-OR¹, (C₁-C₆)-Alkyl-CON(R¹)₂, (C₁-C₆)-Alkyl-SO₂N(R¹)₂, (C₁-C₆)-Alkyl-NR¹COR¹, (C₁-C₆)-Alkyl-NR¹SO₂R², (C₁-C₆)-Alkyl-Phenyl, (C₁-C₆)-Alkyl-Heteroaryl, (C₁-C₆)-Alkyl-Heterocyclyl, Phenyl, Heteroaryl oder Heterocyclyl, wobei die letzten 6 Reste jeweils durch s Reste aus der Gruppe Halogen, Nitro, Cyano, (C₁-C₆)-Alkyl, Halogen-(C₁-C₆)-alkyl, (C₃-C₆)-Cycloalkyl, S(O)ₙ-(C₁-C₆)-Alkyl, (C₁-C₆)-Alkoxy, Halogen-(C₁-C₆)-alkoxy, (C₁-C₆)-Alkoxy-(C₁-C₄)-alkyl und Cyanomethyl substituiert sind, und wobei Heterocyclyl n Oxogruppen trägt,
Z bedeutet Halogen, Cyano, Nitro, Methyl, Halogen-(C₁-C₆)-alkyl, (C₃-C₆)-Cycloalkyl, S(O)ₙR², 1,2,4-Triazol-1-yl, Pyrazol-1-yl, oder
Z kann auch Wasserstoff bedeuten, falls Y für den Rest S(O)ₙR² steht,
R¹ bedeutet Wasserstoff, (C₁-C₆)-Alkyl, (C₂-C₆)-Alkenyl, (C₂-C₆)-Alkinyl, (C₃-C₆)-Cycloalkyl, (C₃-C₆)-Cycloalkyl-(C₁-C₆)-alkyl, (C₁-C₆)-Alkyl-O-(C₁-C₆)-alkyl, Phenyl, Phenyl-(C₁-C₆)-alkyl, Heteroaryl, (C₁-C₆)-Alkyl-Heteroaryl, Heterocyclyl, (C₁-C₆)-Alkyl-Heterocyclyl, (C₁-C₆)-Alkyl-O-Heteroaryl, (C₁-C₆)-Alkyl-O-Heterocyclyl, (C₁-C₆)-Alkyl-NR³-Heteroaryl oder (C₁-C₆)-Alkyl-NR³-Heterocyclyl, wobei die 16 letztgenannten Reste durch s Reste aus der Gruppe bestehend aus Cyano, Halogen, Nitro, OR³, S(O)ₙR⁴, N(R³)₂, NR³OR³, COR³, OCOR³, NR³COR³, NR³SO₂R⁴, CO₂R³, CON(R³)₂ und (C₁-C₄)-Alkoxy-(C₂-C₆)-alkoxycarbonyl substituiert sind, und wobei Heterocyclyl n Oxogruppen trägt,
R² bedeutet (C₁-C₆)-Alkyl, (C₃-C₆)-Cycloalkyl oder (C₃-C₆)-Cycloalkyl-(C₁-C₆)-alkyl, wobei diese drei vorstehend genannten Reste jeweils durch s Reste aus der Gruppe bestehend aus Halogen und OR³ substituiert sind,
R³ bedeutet Wasserstoff oder (C₁-C₆)-Alkyl,
R⁴ bedeutet (C₁-C₆)-Alkyl,
n bedeutet 0, 1 oder 2,
s bedeutet 0, 1, 2 oder 3,
M+ bedeutet ein Kation ausgewählt aus der Gruppe bestehend aus Natrium-Ion, Kalium-Ion, Lithium-Ion, Magnesium-Ion, Calcium-Ion, NH₄⁺-Ion, (2-Hydroxyeth-1-yl)ammonium-lon, Bis-N,N-(2-hydroxyeth-1-yl)-ammonium-lon, Tris-N,N,N-(2-hydroxyeth-1-yl)ammonium-lon, Tetra-N,N,N,N-(2-hydroxyeth-1-yl)ammonium-Ion, N-(2-Hydroxyeth-1-yl)-tris-N,N,N-methylammonium-Ion, Methylammonium-Ion, Dimethylammonium-Ion, Trimethylammonium-Ion, Tetramethylammonium-Ion, Ethylammonium-Ion, Diethylammonium-Ion, Triethylammonium-Ion, Tetraethylammonium-Ion, Isopropylammonium-Ion, Diisopropylammonium-Ion, Tetrapropylammonium-Ion, Tetrabutyl-ammonium-Ion, Tetraoctylammonium-Ion; 2-(2-Hydroxyeth-1-oxy)eth-1-yl-ammonium-Ion, Di-(2-hydroxy-eth-1-yl)-ammonium-Ion, Trimethylbenzylammonium-Ion, Triethylbenzylammonium-Ion, Tri-((C1-C4)-alkyl)-sulfonium-Ion, Benzylammonium-Ion, 1-Phenylethylammonium-Ion, 2-Phenylethylammonium-lon, Diisopropylethylammonium-Ion, Pyridinium-Ion, Piperidinium-Ion, Imidazolium-Ion, Morpholinium-Ion, 1,8-Diazabicyclo-[5.4.0]undec-7-enium-Ion.

Die Erfindung bezieht sich auf Verbindungen der Formel (I), worin A bedeutet N oder CY,
R bedeutet C₁-C₆)-Alkyl, (C₃-C₇)-Cycloalkyl oder
Methoxymethyl,
X bedeutet Halogen, (C₁-C₆)-Alkyl, Halogen-(C₁-C₆)-alkyl, OR¹ oder S(O)ₙR²,
Y (C₁-C₆)-Alkyl, (C₁-C₆)-Halogenalkyl, OR¹ oder S(O)ₙR²,
Z bedeutet Halogen, Methyl, Halogen-(C₁-C₆)-alkyl oder S(O)ₙR²,
R¹ bedeutet Wasserstoff, (C₁-C₆)-Alkyl oder (C₃-C₆)-Cycloalkyl,
R² bedeutet (C₁-C₆)-Alkyl,
n bedeutet 0, 1 oder 2,

M+ bedeutet ein Kation ausgewählt aus der Gruppe bestehend aus Natrium-Ion, Kalium-Ion, Lithium-Ion, Magnesium-Ion und Calcium-Ion.

Weitere erfindungsgemäße Verbindungen werden in Anspruch 2 beschrieben.

Erfindungsgemäße Verbindungen können beispielsweise nach der in Schema 1 angegebenen Methode durch Deprotonierung eines N-(1,3,4-Oxadiazol-2-yl)benzamides und -Nicotinamides (II) mit einer geeigneten Base der Formel M⁺B⁻ (Schema 1), wobei B⁻ zum Beispiel Hydrid, Hydroxy- oder Alkoxyanionen, wie M ethoxy, Ethoxy, n-Propoxy, i-Propoxy, n-Butoxy oder t-Butoxy, hergestellt werden.

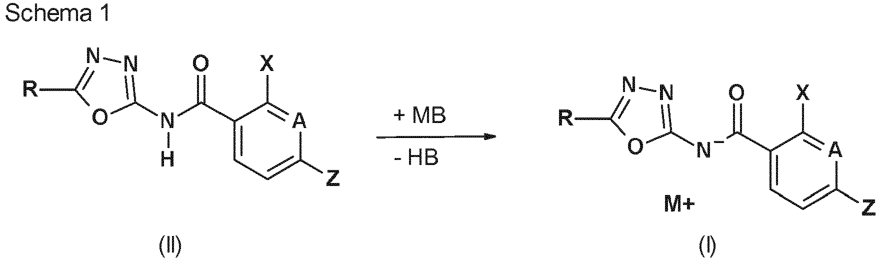

Die Verbindungen der Formel (II) sind aus WO 2012/126932 A1 bekannt und können beispielsweise nach den dort beschriebenen Methoden hergestellt werden.

Die erfindungsgemäßen Verbindungen der Formel (I) weisen eine ausgezeichnete herbizide Wirksamkeit gegen ein breites Spektrum wirtschaftlich wichtiger mono- und dikotyler annueller Schadpflanzen auf. Auch schwer bekämpfbare perennierende Schadpflanzen, die aus Rhizomen, Wurzelstöcken oder anderen Dauerorganen austreiben, werden durch die Wirkstoffe gut erfaßt.

Gegenstand der vorliegenden Erfindung ist daher auch ein Verfahren zur Bekämpfung von unerwünschten Pflanzen oder zur Wachstumsregulierung von Pflanzen, vorzugsweise in Pflanzenkulturen, worin eine oder mehrere erfindungsgemäße Verbindung(en) auf die Pflanzen (z.B. Schadpflanzen wie mono- oder dikotyle Unkräuter oder unerwünschte Kulturpflanzen), das Saatgut (z.B. Körner, Samen oder vegetative Vermehrungsorgane wie Knollen oder Sprossteile mit Knospen) oder die Fläche, auf der die Pflanzen wachsen (z.B. die Anbaufläche), ausgebracht werden. Dabei können die erfindungsgemäßen Verbindungen z.B. im Vorsaat- (ggf. auch durch Einarbeitung in den Boden), Vorauflauf- oder Nachauflaufverfahren ausgebracht werden. Im einzelnen seien beispielhaft einige Vertreter der mono- und dikotylen Unkrautflora genannt, die durch die erfindungsgemäßen Verbindungen kontrolliert werden können, ohne dass durch die Nennung eine Beschränkung auf bestimmte Arten erfolgen soll.

Monokotyle Schadpflanzen der Gattungen: Aegilops, Agropyron, Agrostis, Alopecurus, Apera, Avena, Brachiaria, Bromus, Cenchrus, Commelina, Cynodon, Cyperus, Dactyloctenium, Digitaria, Echinochloa, Eleocharis, Eleusine, Eragrostis, Eriochloa, Festuca, Fimbristylis, Heteranthera, Imperata, Ischaemum, Leptochloa, Lolium, Monochoria, Panicum, Paspalum, Phalaris, Phleum, Poa, Rottboellia, Sagittaria, Scirpus, Setaria, Sorghum.

Dikotyle Unkräuter der Gattungen: Abutilon, Amaranthus, Ambrosia, Anoda, Anthemis, Aphanes, Artemisia, Atriplex, Bellis, Bidens, Capsella, Carduus, Cassia, Centaurea, Chenopodium, Cirsium, Convolvulus, Datura, Desmodium, Emex, Erysimum, Euphorbia, Galeopsis, Galinsoga, Galium, Hibiscus, Ipomoea, Kochia, Lamium, Lepidium, Lindernia, Matricaria, Mentha, Mercurialis, Mullugo, Myosotis, Papaver, Pharbitis, Plantago, Polygonum, Portulaca, Ranunculus, Raphanus, Rorippa, Rotala, Rumex, Salsola, Senecio, Sesbania, Sida, Sinapis, Solanum, Sonchus, Sphenoclea, Stellaria, Taraxacum, Thlaspi, Trifolium, Urtica, Veronica, Viola, Xanthium.

Werden die erfindungsgemäßen Verbindungen vor dem Keimen auf die Erdoberfläche appliziert, so wird entweder das Auflaufen der Unkrautkeimlinge vollständig verhindert oder die Unkräuter wachsen bis zum Keimblattstadium heran, stellen jedoch dann ihr Wachstum ein und sterben schließlich nach Ablauf von drei bis vier Wochen vollkommen ab.

Bei Applikation der Wirkstoffe auf die grünen Pflanzenteile im Nachauflaufverfahren tritt nach der Behandlung Wachstumsstop ein und die Schadpflanzen bleiben in dem zum Applikationszeitpunkt vorhandenen Wachstumsstadium stehen oder sterben nach einer gewissen Zeit ganz ab, so dass auf diese Weise eine für die Kulturpflanzen schädliche Unkrautkonkurrenz sehr früh und nachhaltig beseitigt wird.

Obgleich die erfindungsgemäßen Verbindungen eine ausgezeichnete herbizide Aktivität gegenüber mono- und dikotylen Unkräutern aufweisen, werden Kulturpflanzen wirtschaftlich bedeutender Kulturen z.B. dikotyler Kulturen der Gattungen Arachis, Beta, Brassica, Cucumis, Cucurbita, Helianthus, Daucus, Glycine, Gossypium, Ipomoea, Lactuca, Linum, Lycopersicon, Nicotiana, Phaseolus, Pisum, Solanum, Vicia, oder monokotyler Kulturen der Gattungen Allium, Ananas, Asparagus, Avena, Hordeum, Oryza, Panicum, Saccharum, Secale, Sorghum, Triticale, Triticum, Zea, insbesondere Zea und Triticum, abhängig von der Struktur der jeweiligen erfindungsgemäßen Verbindung und deren Aufwandmenge nur unwesentlich oder gar nicht geschädigt. Die vorliegenden Verbindungen eignen sich aus diesen Gründen sehr gut zur selektiven Bekämpfung von unerwünschtem Pflanzenwuchs in Pflanzenkulturen wie landwirtschaftlichen Nutzpflanzungen oder Zierpflanzungen.

Darüberhinaus weisen die erfindungsgemäßen Verbindungen (abhängig von ihrer jeweiligen Struktur und der ausgebrachten Aufwandmenge) hervorragende wachstumsregulatorische Eigenschaften bei Kulturpflanzen auf. Sie greifen regulierend in den pflanzeneigenen Stoffwechsel ein und können damit zur gezielten Beeinflussung von Pflanzeninhaltsstoffen und zur Ernteerleichterung wie z.B. durch Auslösen von Desikkation und Wuchsstauchung eingesetzt werden. Desweiteren eignen sie sich auch zur generellen Steuerung und Hemmung von unerwünschtem vegetativen Wachstum, ohne dabei die Pflanzen abzutöten. Eine Hemmung des vegetativen Wachstums spielt bei vielen mono- und dikotylen Kulturen eine große Rolle, da beispielsweise die Lagerbildung hierdurch verringert oder völlig verhindert werden kann.

Aufgrund ihrer herbiziden und pflanzenwachstumsregulatorischen Eigenschaften können die Wirkstoffe auch zur Bekämpfung von Schadpflanzen in Kulturen von gentechnisch oder durch konventionelle Mutagenese veränderten Pflanzen eingesetzt werden. Die transgenen Pflanzen zeichnen sich in der Regel durch besondere vorteilhafte Eigenschaften aus, beispielsweise durch Resistenzen gegenüber bestimmten Pestiziden, vor allem bestimmten Herbiziden, Resistenzen gegenüber Pflanzenkrankheiten oder Erregern von Pflanzenkrankheiten wie bestimmten Insekten oder Mikroorganismen wie Pilzen, Bakterien oder Viren. Andere besondere Eigenschaften betreffen z. B. das Erntegut hinsichtlich Menge, Qualität, Lagerfähigkeit, Zusammensetzung und spezieller Inhaltsstoffe. So sind transgene Pflanzen mit erhöhtem Stärkegehalt oder veränderter Qualität der Stärke oder solche mit anderer Fettsäurezusammensetzung des Ernteguts bekannt.

Bevorzugt bezüglich transgener Kulturen ist die Anwendung der erfindungsgemäßen Verbindungen in wirtschaftlich bedeutenden transgenen Kulturen von Nutz- und Zierpflanzen, z. B. von Getreide wie Weizen, Gerste, Roggen, Hafer, Hirse, Reis und Mais oder auch Kulturen von Zuckerrübe, Baumwolle, Soja, Raps, Kartoffel, Tomate, Erbse und anderen Gemüsesorten.Vorzugsweise können die erfindungsgemäßen Verbindungen als Herbizide in Nutzpflanzenkulturen eingesetzt werden, welche gegenüber den phytotoxischen Wirkungen der Herbizide resistent sind bzw. gentechnisch resistent gemacht worden sind.

Bevorzugt ist die Anwendung der erfindungsgemäßen Verbindungen in wirtschaftlich bedeutenden transgenen Kulturen von Nutz-und Zierpflanzen, z. B. von Getreide wie Weizen, Gerste, Roggen, Hafer, Hirse, Reis, Maniok und Mais oder auch Kulturen von Zuckerrübe, Baumwolle, Soja, Raps, Kartoffel, Tomate, Erbse und anderen Gemüsesorten. Vorzugsweise können die erfindungsgemäßen Verbindungen als Herbizide in Nutzpflanzenkulturen eingesetzt werden, welche gegenüber den phytotoxischen Wirkungen der Herbizide resistent sind bzw. gentechnisch resistent gemacht worden sind.

Herkömmliche Wege zur Herstellung neuer Pflanzen, die im Vergleich zu bisher vorkommenden Pflanzen modifizierte Eigenschaften aufweisen, bestehen beispielsweise in klassischen Züchtungsverfahren und der Erzeugung von Mutanten. Alternativ können neue Pflanzen mit veränderten Eigenschaften mit Hilfe gentechnischer Verfahren erzeugt werden (siehe z. B. EP-A-0221044, EP-A-0131624). Beschrieben wurden beispielsweise in mehreren Fällen
- gentechnische Veränderungen von Kulturpflanzen zwecks Modifikation der in den Pflanzen synthetisierten Stärke (z. B. WO 92/11376, WO 92/14827, WO 91/19806),
- transgene Kulturpflanzen, welche gegen bestimmte Herbizide vom Typ Glufosinate (vgl. z. B. EP-A-0242236, EP-A-242246) oder Glyphosate (WO 92/00377) oder der Sulfonylharnstoffe (EP-A-0257993, US-A-5013659) resistent sind,
- transgene Kulturpflanzen, beispielsweise Baumwolle, mit der Fähigkeit Bacillus thuringiensis-Toxine (Bt-Toxine) zu produzieren, welche die Pflanzen gegen bestimmte Schädlinge resistent machen (EP-A-0142924, EP-A-0193259).
- transgene Kulturpflanzen mit modifizierter Fettsäurezusammensetzung (WO 91/13972).
- gentechnisch veränderte Kulturpflanzen mit neuen Inhalts- oder Sekundärstoffen z. B. neuen Phytoalexinen, die eine erhöhte Krankheitsresistenz verursachen (EPA 309862, EPA0464461)
- gentechnisch veränderte Pflanzen mit reduzierter Photorespiration, die höhere Erträge und höhere Stresstoleranz aufweisen (EPA 0305398).
- Transgene Kulturpflanzen, die pharmazeutisch oder diagnostisch wichtige Proteine produzieren ("molecular pharming")
- transgene Kulturpflanzen, die sich durch höhere Erträge oder bessere Qualitat auszeichnen
- transgene Kulturpflanzen die sich durch eine Kombinationen z. B. der o. g. neuen Eigenschaften auszeichnen ("gene stacking")

Zahlreiche molekularbiologische Techniken, mit denen neue transgene Pflanzen mit veränderten Eigenschaften hergestellt werden können, sind im Prinzip bekannt, siehe z. B. I. Potrykus und G. Spangenberg (eds.) Gene Transfer to Plants, Springer Lab Manual (1995), Springer Verlag Berlin, Heidelberg. oder Christou, "Trends in Plant Science" 1 (1996) 423-431).

Für derartige gentechnische Manipulationen können Nucleinsäuremoleküle in Plasmide eingebracht werden, die eine Mutagenese oder eine Sequenzveränderung durch Rekombination von DNA-Sequenzen erlauben. Mit Hilfe von Standardverfahren können z. B. Basenaustausche vorgenommen, Teilsequenzen entfernt oder natürliche oder synthetische Sequenzen hinzugefügt werden. Für die Verbindung der DNA-Fragmente untereinander können an die Fragmente Adaptoren oder Linker angesetzt werden, siehe z. B. Sambrook et al., 1989, Molecular Cloning, A Laboratory Manual, 2. Aufl. Cold Spring Harbor Laboratory Press, Cold Spring Harbor, NY, oder Winnacker "Gene und Klone", VCH Weinheim 2. Auflage 1996

Die Herstellung von Pflanzenzellen mit einer verringerten Aktivität eines Genprodukts kann beispielsweise erzielt werden durch die Expression mindestens einer entsprechenden antisense-RNA, einer sense-RNA zur Erzielung eines Cosuppressionseffektes oder die Expression mindestens eines entsprechend konstruierten Ribozyms, das spezifisch Transkripte des obengenannten Genprodukts spaltet. Hierzu können zum einen DNA-Moleküle verwendet werden, die die gesamte codierende Sequenz eines Genprodukts einschließlich eventuell vorhandener flankierender Sequenzen umfassen, als auch DNA-Moleküle, die nur Teile der codierenden Sequenz umfassen, wobei diese Teile lang genug sein müssen, um in den Zellen einen antisense-Effekt zu bewirken. Möglich ist auch die Verwendung von DNA-Sequenzen, die einen hohen Grad an Homologie zu den codiereden Sequenzen eines Genprodukts aufweisen, aber nicht vollkommen identisch sind.

Bei der Expression von Nucleinsäuremolekülen in Pflanzen kann das synthetisierte Protein in jedem beliebigen Kompartiment der pflanzlichen Zelle lokalisiert sein. Um aber die Lokalisation in einem bestimmten Kompartiment zu erreichen, kann z. B. die codierende Region mit DNA-Sequenzen verknüpft werden, die die Lokalisierung in einem bestimmten Kompartiment gewährleisten. Derartige Sequenzen sind dem Fachmann bekannt (siehe beispielsweise Braun et al., EMBO J. 11 (1992), 3219-3227, Wolter et al., Proc. Natl. Acad. Sci. USA 85 (1988), 846-850, Sonnewald et al., Plant J. 1 (1991), 95-106). Die Expression der Nukleinsäuremoleküle kann auch in den Organellen der Pflanzenzellen stattfinden.

Die transgenen Pflanzenzellen können nach bekannten Techniken zu ganzen Pflanzen regeneriert werden. Bei den transgenen Pflanzen kann es sich prinzipiell um Pflanzen jeder beliebigen Pflanzenspezies handeln, d.h., sowohl monokotyle als auch dikotyle Pflanzen.

So sind transgene Pflanzen erhältlich, die veränderte Eigenschaften durch Überexpression, Suppression oder Inhibierung homologer (= natürlicher) Gene oder Gensequenzen oder Expression heterologer (= fremder) Gene oder Gensequenzen aufweisen.

Vorzugsweise können die erfindungsgemäßen Verbindungen in transgenen Kulturen eingesetzt werden, welche gegen Wuchsstoffe, wie z. B. Dicamba oder gegen Herbizide, die essentielle Pflanzenenzyme, z. B. Acetolactatsynthasen (ALS), EPSP Synthasen, Glutaminsynthasen (GS) oder Hydroxyphenylpyruvat Dioxygenasen (HPPD) hemmen, respektive gegen Herbizide aus der Gruppe der Sulfonylharnstoffe, der Glyphosate, Glufosinate oder Benzoylisoxazole und analogen Wirkstoffe, resistent sind.

Bei der Anwendung der erfindungsgemäßen Wirkstoffe in transgenen Kulturen treten neben den in anderen Kulturen zu beobachtenden Wirkungen gegenüber Schadpflanzen oftmals Wirkungen auf, die für die Applikation in der jeweiligen transgenen Kultur spezifisch sind, beispielsweise ein verändertes oder speziell erweitertes Unkrautspektrum, das bekämpft werden kann, veränderte Aufwandmengen, die für die Applikation eingesetzt werden können, vorzugsweise gute Kombinierbarkeit mit den Herbiziden, gegenüber denen die transgene Kultur resistent ist, sowie Beeinflussung von Wuchs und Ertrag der transgenen Kulturpflanzen.

Gegenstand der Erfindung ist deshalb auch die Verwendung der erfindungsgemäßen Verbindungen als Herbizide zur Bekämpfung von Schadpflanzen in transgenen Kulturpflanzen.

Die erfindungsgemäßen Verbindungen zeigen verglichen zu ihren korrespendierenden Säuren eine höhere Löslichkeit in Wasser und damit beispielsweise vorteilhaftere Formuliereigenschaften. Sie eignen sich sehr gut zur Herstellung von Wasser basierten Formulierungen.

Die erfindungsgemäßen Verbindungen können in Form von Spritzpulvern, emulgierbaren Konzentraten, versprühbaren Lösungen, Stäubemitteln oder Granulaten in den üblichen Zubereitungen angewendet werden. Gegenstand der Erfindung sind deshalb auch herbizide und pflanzenwachstumsregulierende Mittel, welche die erfindungsgemäßen Verbindungen enthalten.

Die erfindungsgemäßen Verbindungen können auf verschiedene Art formuliert werden, je nachdem welche biologischen und/oder chemisch-physikalischen Parameter vorgegeben sind. Als Formulierungsmöglichkeiten kommen beispielsweise in Frage: Spritzpulver (WP), wasserlösliche Pulver (SP), wasserlösliche Konzentrate, emulgierbare Konzentrate (EC), Emulsionen (EW), wie Öl-in-Wasser- und Wasser-in-ÖI-Emulsionen, versprühbare Lösungen, Suspensionskonzentrate (SC), Dispersionen auf Öl- oder Wasserbasis, ölmischbare Lösungen, Kapselsuspensionen (CS), Stäubemittel (DP), Beizmittel, Granulate für die Streu- und Bodenapplikation, Granulate (GR) in Form von Mikro-, Sprüh-, Aufzugs- und Adsorptionsgranulaten, wasserdispergierbare Granulate (WG), wasserlösliche Granulate (SG), ULV-Formulierungen, Mikrokapseln und Wachse. Diese einzelnen Formulierungstypen sind im Prinzip bekannt und werden beispielsweise beschrieben in: Winnacker-Küchler, "Chemische Technologie", Band 7, C. Hanser Verlag München, 4. Aufl. 1986, Wade van Valkenburg, "Pesticide Formulations", Marcel Dekker, N.Y., 1973, K. Martens, "Spray Drying" Handbook, 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J., H.v. Olphen, "Introduction to Clay Colloid Chemistry", 2nd Ed., J. Wiley & Sons, N.Y., C. Marsden, "Solvents Guide", 2nd Ed., Interscience, N.Y. 1963, McCutcheon's "Detergents and Emulsifiers Annual", MC Publ. Corp., Ridgewood N.J., Sisley and Wood, "Encyclopedia of Surface Active Agents", Chem. Publ. Co. Inc., N.Y. 1964, Schönfeldt, "Grenzflächenaktive Äthylenoxidaddukte", Wiss. Verlagsgesell., Stuttgart 1976, Winnacker-Küchler, "Chemische Technologie", Band 7, C. Hanser Verlag München, 4. Aufl. 1986.

Auf der Basis dieser Formulierungen lassen sich auch Kombinationen mit anderen pestizid wirksamen Stoffen, wie z.B. Insektiziden, Akariziden, Herbiziden, Fungiziden, sowie mit Safenern, Düngemitteln und/oder Wachstumsregulatoren herstellen, z.B. in Form einer Fertigformulierung oder als Tankmix. Geeignete Safener sind z.B. Mefenpyr-diethyl, Cyprosulfamid, Isoxadifen-ethyl, Cloquintocet-mexyl und Dichlormid.

Spritzpulver sind in Wasser gleichmäßig dispergierbare Präparate, die neben dem Wirkstoff außer einem Verdünnungs- oder Inertstoff noch Tenside ionischer und/oder nichtionischer Art (Netzmittel, Dispergiermittel), z.B. polyoxyethylierte Alkylphenole, polyoxethylierte Fettalkohole, polyoxethylierte Fettamine, Fettalkoholpolyglykolethersulfate, Alkansulfonate, Alkylbenzolsulfonate, ligninsulfonsaures Natrium, 2,2'-dinaphthylmethan-6,6'-disulfonsaures Natrium, dibutylnaphthalin-sulfonsaures Natrium oder auch oleoylmethyltaurinsaures Natrium enthalten. Zur Herstellung der Spritzpulver werden die herbiziden Wirkstoffe beispielsweise in üblichen Apparaturen wie Hammermühlen, Gebläsemühlen und Luftstrahlmühlen feingemahlen und gleichzeitig oder anschließend mit den Formulierungshilfsmitteln vermischt.

Emulgierbare Konzentrate werden durch Auflösen des Wirkstoffes in einem organischen Lösungsmittel z.B. Butanol, Cyclohexanon, Dimethylformamid, Xylol oder auch höhersiedenden Aromaten oder Kohlenwasserstoffen oder Mischungen der organischen Lösungsmittel unter Zusatz von einem oder mehreren Tensiden ionischer und/oder nichtionischer Art (Emulgatoren) hergestellt. Als Emulgatoren können beispielsweise verwendet werden: Alkylarylsulfonsaure Calzium-Salze wie Ca-Dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanester wie z.B. Sorbitanfettsäureester oder Polyoxethylensorbitanester wie z.B. Polyoxyethylensorbitanfettsäureester.

Stäubemittel erhält man durch Vermahlen des Wirkstoffes mit fein verteilten festen Stoffen, z.B. Talkum, natürlichen Tonen, wie Kaolin, Bentonit und Pyrophyllit, oder Diatomeenerde.

Suspensionskonzentrate können auf Wasser- oder Ölbasis sein. Sie können beispielsweise durch Naß-Vermahlung mittels handelsüblicher Perlmühlen und gegebenenfalls Zusatz von Tensiden, wie sie z.B. oben bei den anderen Formulierungstypen bereits aufgeführt sind, hergestellt werden.

Emulsionen, z.B. Öl-in-Wasser-Emulsionen (EW), lassen sich beispielsweise mittels Rührern, Kolloidmühlen und/oder statischen Mischern unter Verwendung von wäßrigen organischen Lösungsmitteln und gegebenenfalls Tensiden, wie sie z.B. oben bei den anderen Formulierungstypen bereits aufgeführt sind, herstellen.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

Wasserdispergierbare Granulate werden in der Regel nach den üblichen Verfahren wie Sprühtrocknung, Wirbelbett-Granulierung, Teller-Granulierung, Mischung mit Hochgeschwindigkeitsmischern und Extrusion ohne festes Inertmaterial hergestellt.

Zur Herstellung von Teller-, Fließbett-, Extruder- und Sprühgranulate siehe z.B. Verfahren in "Spray-Drying Handbook" 3rd ed. 1979, G. Goodwin Ltd., London, J.E. Browning, "Agglomeration", Chemical and Engineering 1967, Seiten 147 ff, "Perry's Chemical Engineer's Handbook", 5th Ed., McGraw-Hill, New York 1973, S. 8-57.

Für weitere Einzelheiten zur Formulierung von Pflanzenschutzmitteln siehe z.B. G.C. Klingman, "Weed Control as a Science", John Wiley and Sons, Inc., New York, 1961, Seiten 81-96 und J.D. Freyer, S.A. Evans, "Weed Control Handbook", 5th Ed., Blackwell Scientific Publications, Oxford, 1968, Seiten 101-103.

Die agrochemischen Zubereitungen enthalten in der Regel 0.1 bis 99 Gew.-%, insbesondere 0.1 bis 95 Gew.-%, erfindungsgemäße Verbindungen.
In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 90, vorzugsweise 5 bis 80 Gew.-% betragen. Staubförmige Formulierungen enthalten 1 bis 30 Gew.-% Wirkstoff, vorzugsweise meistens 5 bis 20 Gew.-% an Wirkstoff, versprühbare Lösungen enthalten etwa 0.05 bis 80, vorzugsweise 2 bis 50 Gew.-% Wirkstoff. Bei wasserdispergierbaren Granulaten hängt der Wirkstoffgehalt zum Teil davon ab, ob die wirksame Verbindung flüssig oder fest vorliegt und welche Granulierhilfsmittel, Füllstoffe usw. verwendet werden. Bei den in Wasser dispergierbaren Granulaten liegt der Gehalt an Wirkstoff beispielsweise zwischen 1 und 95 Gew.-%, vorzugsweise zwischen 10 und 80 Gew.-%.

Daneben enthalten die genannten Wirkstofformulierungen gegebenenfalls die jeweils üblichen Haft-, Netz-, Dispergier-, Emulgier-, Penetrations-, Konservierungs-, Frostschutz- und Lösungsmittel, Füll-, Träger- und Farbstoffe, Entschäumer, Verdunstungshemmer und den pH-Wert und die Viskosität beeinflussende Mittel.

Auf der Basis dieser Formulierungen lassen sich auch Kombinationen mit anderen pestizid wirksamen Stoffen, wie z.B. Insektiziden, Akariziden, Herbiziden, Fungiziden, sowie mit Safenern, Düngemitteln und/oder Wachstumsregulatoren herstellen, z.B. in Form einer Fertigformulierung oder als Tankmix.

Zur Anwendung werden die in handelsüblicher Form vorliegenden Formulierungen gegebenenfalls in üblicher Weise verdünnt z.B. bei Spritzpulvern, emulgierbaren Konzentraten, Dispersionen und wasserdispergierbaren Granulaten mittels Wasser. Staubförmige Zubereitungen, Boden- bzw. Streugranulate sowie versprühbare Lösungen werden vor der Anwendung üblicherweise nicht mehr mit weiteren inerten Stoffen verdünnt.

Mit den äußeren Bedingungen wie Temperatur, Feuchtigkeit, der Art des verwendeten Herbizids, u.a. variiert die erforderliche Aufwandmenge der Verbindungen der Formel (I). Sie kann innerhalb weiter Grenzen schwanken, z.B. zwischen 0,001 und 1,0 kg/ha oder mehr Aktivsubstanz, vorzugsweise liegt sie jedoch zwischen 0,005 und 750 g/ha.

Die nachstehenden Beispiele erläutern die Erfindung.

### A. Chemische Beispiele

### Herstellung des Natriumsalzes von 2-Methyl-N-(5-methyl-1,3,4-oxadiazol-2-yl)-3-(methylsulfonyl)-4-(trifluormethyl)benzamid (Nr. 1-14)

Zu einer Lösung von 200 mg (0,55 mmol) 2-Methyl-N-(5-methyl-1,3,4-oxadiazol-2-yl)-3-(methylsulfonyl)-4-(trifluormethyl)benzamid in 5 ml Methanol wurden bei Raumtemperatur (RT) 0,101 ml (0,55 mmol) einer 30%igen Natriummethylat-Lösung in Methanol zugegeben. Nach 8 h Rühren bei RT wurde eingeengt. Der Rückstand wurde zweimal mit 5 ml abs. Toluol versetzt und bis zur Trockne eingeengt. Ausbeute: 0,21 g (0,55 mmol; 99%).
¹H-NMR (DMSO-d₆, 400 MHz): 7.79 (d,1H), 7.67 (d,1H), 3.34 (s; 3H); 2.71 (s,3H), 2.28 (s, 3H).

### Herstellung des Beta-hydroxyethyltrimethylammonium-Salzes von 2-Chlor-N-(5-methyl-1,3,4-oxadiazol-2-yl)-3-(methylsulfinyl)-4-(trifluormethyl)benzamid (Nr. 1-153)

Zu einer Lösung von 100 mg (0,272 mmol) 2-Chlor-N-(5-methyl-1,3,4-oxadiazol-2-yl)-3-(methylsulfinyl)-4-(trifluormethyl)benzamid in 2,5 ml Methanol wurde bei RT eine Lösung von 0,077 ml (0,272 mmol) einer 45 %igen Lösung von Beta-hydroxyethyltrimethylammonium-hydroxid zugegeben. Nach 8 h Rühren bei RT wurde eingeengt. Der Rückstand wurde zweimal mit 5 ml Toluol versetzt und bis zur Trockne eingeengt. Ausbeute: 0,12 g (0,25 mmol; 94%).
¹H-NMR (DMSO-d₆, 400 MHz): 7.74 (d,1H), 7.65 (d,1H), 4.06 (bs, 2H); 3.72 (bs, 2H); 3.28 (s, 9H); 3.11 (s,3H); 2.38 (s, 3H).

Die in den nachfolgenden Tabellen aufgeführten Beispiele wurden analog oben genannten Methoden hergestellt beziehungsweise sind analog oben genannten Methoden erhältlich. Die in den nachfolgenden Tabellen aufgeführten Verbindungen sind ganz besonders bevorzugt. Insbesonders ganz besonders bevorzugt sind Salze von N-(1,3,4-Oxadiazol-2-yl)benzamiden gemäß Formel (I), worin A bedeutet CY,
R bedeutet Methyl,
X bedeutet Methyl,
Y Methylsulfonyl,
Z Trifluormethyl,
M+ bedeutet ein Kation ausgewählt aus der Gruppe bestehend aus Natrium-Ion, Kalium-Ion, N-(2-Hydroxyeth-1-yl)-tris-N,N,N-methylammonium-lon, Tetramethylammonium-Ion, Tetrapropylammonium-Ion, Tetraoctylammonium-Ion, Trimethylbenzylammonium-Ion.

Die hier verwendeten Abkürzungen bedeuten:

| | | | | | |
|---|---|---|---|---|---|
| Ac | = Acetyl | Bn | = Benzyl | Bu | = n-Butyl |
| c-Pr | = c-Propyl | Et | = Ethyl | Me | = Methyl |
| n-Oct | = n-Octyl | Pr | = n-Propyl | | |

**Tabelle 1: Verbindungen der allgemeinen Formel (I), worin A für CY steht**

| | | | | | | |
|---|---|---|---|---|---|---|
| 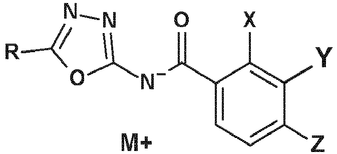 | | | | | | |

| **Nr.** | **R** | **X** | **Y** | **Z** | **M⁺** | **Physikalische Daten** |
|---|---|---|---|---|---|---|
| | | | | | | **(¹H-NMR, DMSO-d₆, 400 MHz)** |
| 1-1 | Me | Cl | H | SO₂Me | Na⁺ | |
| 1-2 | Me | SO₂Me | H | CF3 | Na⁺ | |
| 1-3 | Me | SO₂Me | H | CF₃ | Pr₄N⁺ | |
| 1-4 | Me | SO₂Me | H | CF₃ | Me₃N(CH₂CH₂OH)⁺ | |
| 1-5 | Me | SMe | SMe | CF₃ | Na⁺ | |
| 1-6 | Me | SMe | SOMe | CF₃ | Na⁺ | |
| 1-7 | Me | SMe | SO₂Me | CF₃ | Na⁺ | |
| 1-8 | Me | Me | SMe | CF₃ | Na⁺ | |
| 1-9 | Me | Me | SMe | C₂F₅ | Na⁺ | |
| 1-10 | c-Pr | Me | SMe | CF₃ | Na⁺ | |
| 1-11 | Me | Me | SOMe | CF₃ | Na⁺ | |
| 1-12 | Me | Me | SOMe | CF₃ | Me₃N(CH₂CH₂OH)⁺ | |
| 1-13 | Et | Me | SOMe | CF₃ | Na⁺ | |
| 1-14 | Me | Me | SO₂Me | CF₃ | Na⁺ | 7.79 (d,1H), 7.67 (d,1H), 3.34 (s; 3H); 2.71 (s, 3H), 2.28 (s, 3H) |
| 1-15 | Me | Me | SO₂Me | CF₃ | Li⁺ | 7.83 (d,1H), 7.74 (d,1H), 3.34 (s; 3H); 2.72 (s,3H), 2.33 (s, 3H) |
| 1-16 | Me | Me | SO₂Me | CF₃ | K⁺ | 7.78 (d,1H), 7.66 (d,1H), 3.36 (s; 3H); 2.71 (s,3H), 2.28 (s, 3H) |
| 1-17 | Me | Me | SO₂Me | CF₃ | Mg²⁺ | |
| 1-18 | Me | Me | SO₂Me | CF₃ | Ca²⁺ | |
| 1-19 | Me | Me | SO₂Me | CF₃ | Me₃S⁺ | |
| 1-20 | Me | Me | SO₂Me | CF₃ | Et₃S⁺ | |
| 1-21 | Me | Me | SO₂Me | CF₃ | Me₄N⁺ | 7.77 (d,1H), 7.63 (d,1H), 3.33 (s; 3H); 3.10 (s, 12H), 2.70 (s, 3H), 2.27 (s, 3H) |
| 1-22 | Me | Me | SO₂Me | CF₃ | Et₄N⁺ | 7.81 (d,1H), 7.69 (d,1H), 3.34 (s; 3H); 3.20 (q, 8H), 2.70 (s,3H), 2.30 (s, 3H), 1.15 (t, 12H) |
| 1-23 | Me | Me | SO₂Me | CF₃ | Bu₄N⁺ | |
| 1-24 | Me | Me | SO₂Me | CF₃ | i-Pr₄N⁺ | |
| 1-25 | | | | CF₃ | Et₃N(Bn)⁺ | 7.67 (d,1H), 7.61 (d,1H), 7.49-7.38 (m, 5H); 4.57 (s, |
| | Me | Me | SO₂Me | | | 2H); 3.30 (q, 6H), 3.18 (s,3H); 2.84 (s, 3H); 2.38 (s, 3H); 1.41 (t, 9H) |
| 1-26 | Me | Me | SO₂Me | CF₃ | Pr₄N⁺ | 7.77 (d,1H), 7.70 (d,1H), 3.24-3.19 (m, 11H); 2.90 (s,3H); 2.39 (s, 3H); 1.71-1.65 (m, 8H); 0.96 (t, 12H) |
| 1-27 | Me | Me | SO₂Me | CF₃ | Me₃N(CH₂CH₂OH)⁺ | 7.73 (2d, 2H), 4.00 (bs, 2H); 3.64 - 3.62 (m, 2H); 3.27 (s, 9H); 3.20 (s, 3H), 2.82 (s, 3H); 2.38 (s, 3H);) |
| 1-28 | Me | Me | SO₂Me | CF₃ | Me₃N(Bn)⁺ | 7.71 (2d, 2H), 7.50-7.45 (m, 5H); 4.71 (s, 2H); 3.24 (s,9H), 3.20 (s,3H); 2.79 (s, 3H); 2.43 (s, 3H) |
| 1-29 | Me | Me | SO₂Me | CF₃ | n-Oct₄N⁺ | 7.76 (d,1H), 7.69 (d,1H), 3.27-3.22 (m, 8H); 3.18 (s, 3H); 2.37 (s, 3H); 1.62 (m, 8H); 1.30-1.25 (m, 40H); 0.88 (t, 12H) |
| 1-30 | Me | Me | SOMe | C₂F₅ | Na⁺ | |
| 1-31 | MeOCH₂ | Me | SO₂Me | CF₃ | Na⁺ | |
| 1-32 | MeOCH₂ | Me | SO₂Me | CF₃ | Me₃N(CH₂CH₂OH)⁺ | |
| 1-33 | Pr | Me | SO₂Me | CF₃ | Na⁺ | |
| 1-34 | MeO(CH₂) ₂- | Me | SO₂Me | CF₃ | Na⁺ | |
| 1-35 | Me | Me | SEt | CF₃ | Na⁺ | |
| 1-36 | Et | Me | SEt | CF₃ | Na⁺ | |
| 1-37 | Me | Me | SOEt | CF₃ | Na⁺ | |
| 1-38 | Me | Me | SOEt | CHF₂ | Na⁺ | |
| 1-39 | Me | Me | SO₂Et | CF₃ | Na⁺ | |
| 1-40 | Et | Me | SO₂Et | CF₃ | Na⁺ | |
| 1-41 | Me | Me | SO₂Et | CHF₂ | Na⁺ | |
| 1-42 | Me | Me | 1H-Pyrazol-1-yl | CF₃ | Na⁺ | |
| 1-43 | Me | Me | 1H-Pyrazol-1-yl | CF₃ | Me₃N(CH₂CH₂OH)⁺ | |
| 1-44 | Me | Me | 1H-Pyrazol-1-yl | C₂F₅ | Na⁺ | |
| 1-45 | Me | Me | 4-CF₃-1H-Pyrazol-1-yl | CF₃ | Na⁺ | |
| 1-46 | Me | Me | 4-Me-1H-Pyrazol-1-yl | CF₃ | Na⁺ | |
| 1-47 | Me | Me | 2H-1,2,3-triazol-2-yl | CF₃ | Na⁺ | |
| 1-48 | Me | Me | 2H-1,2,3-triazol-2-yl | C₂F₅ | Na⁺ | |
| 1-49 | Me | Me | 1H-1,2,3-triazol-1-yl | CF₃ | Na⁺ | |
| 1-50 | Me | Me | 1H-1,2,3-triazol-1-yl | C₂F₅ | Na⁺ | |
| 1-51 | Me | Me | 1H-1,2,4-triazol-1-yl | CF₃ | Na⁺ | |
| 1-52 | Me | Me | 1H-1,2,4-triazol-1-yl | C₂F₅ | Na⁺ | |
| 1-53 | Me | Me | SMe | CN | Na⁺ | |
| 1-54 | Me | Me | SOMe | CN | Na⁺ | |
| 1-55 | Me | Me | SO₂Me | CN | Na⁺ | |
| 1-56 | Me | Me | SMe | Cl | Na⁺ | |
| 1-57 | Me | Me | SOMe | Cl | Na⁺ | |
| 1-58 | Me | Me | SO₂Me | Cl | Na⁺ | |
| 1-59 | Me | Me | SEt | Cl | Na⁺ | |
| 1-60 | Me | Me | SOEt | Cl | Na⁺ | |
| 1-61 | Et | Me | SOEt | Cl | Na⁺ | |
| 1-62 | Me | Me | SO₂Et | Cl | Na⁺ | |
| 1-63 | Me | Me | SMe | Br | Na⁺ | |
| 1-64 | Me | Me | SEt | Br | Na⁺ | |
| 1-65 | Me | Me | Ac | SO₂Me | Na⁺ | |
| 1-66 | Me | Me | (CO)-c-Pr | SO₂Me | Na⁺ | |
| 1-67 | Me | Me | C(=NOMe)Me | SO₂Me | Na⁺ | |
| 1-68 | Me | Me | C(=NOEt)Me | SO₂Me | Na⁺ | |
| 1-69 | Me | Me | 5-c-Pr-isoxazol-3-yl | SO₂Me | Na⁺ | |
| 1-70 | Me | Me | 5-Methoxymethyl-1,2-oxazol-3-yl | SO₂Me | Na⁺ | |
| 1-71 | Me | Me | 3-Methyl-4,5-dihydro-1,2-oxazol-5-yl | SO₂Me | Na⁺ | |
| 1-72 | Me | Me | 4,5-Dihydro-1,2-oxazol-3-yl | SO₂Me | Na⁺ | |
| 1-73 | Et | Me | 4,5-Dihydro-1,2-oxazol-3-yl | SO₂Me | Na⁺ | |
| 1-74 | Me | Me | Pyrazol-1-yl | SO₂Me | Na⁺ | |
| 1-75 | MeOCH₂ | Me | Pyrazol-1-yl | SO₂Me | Na⁺ | |
| 1-76 | Me | Me | 4-CF₃-1H-Pyrazol-1-yl | SO₂Me | Na⁺ | |
| 1-77 | Me | Me | 4-Cl-1H-Pyrazol-1-yl | SO₂Me | Na⁺ | 8.29 (s, 1H), 7.92 (s, 1H), 7.90 (d,1H), 7.82 (d,1H), 3.09 (s,3H), 2.50 (s,3H), 2.30 (s, 3H) |
| 1-78 | Me | Me | OMe | SO₂Me | Na+ | |
| 1-79 | Me | Me | SMe | SO₂Me | Na⁺ | |
| 1-80 | Me | Me | SOMe | SO₂Me | Na⁺ | |
| 1-81 | Me | Me | SO₂Me | SO₂Me | Na⁺ | |
| 1-82 | Et | Me | SO₂Me | SO₂Me | Na⁺ | |
| 1-83 | Me | Me | SEt | SO₂Me | Na⁺ | |
| 1-84 | Me | Me | SOEt | SO₂Me | Na⁺ | |
| 1-85 | Me | Me | SO₂Et | SO₂Me | Na⁺ | |
| 1-86 | Me | Me | SO₂Et | SO₂Et | Na⁺ | |
| 1-87 | Et | Me | SO₂Et | SO₂Me | Na⁺ | |
| 1-88 | Me | Me | SCH₂CH₂OMe | SO₂Me | Na⁺ | |
| 1-89 | Me | Me | SOCH₂CH₂OMe | SO₂Me | Na⁺ | |
| 1-90 | Me | Me | SO₂CH₂CH₂OMe | SO₂Me | Na⁺ | |
| 1-91 | Me | Et | SMe | CF₃ | Na⁺ | |
| 1-92 | Me | Et | SOMe | CF₃ | Na⁺ | |
| 1-93 | Me | Et | SO₂Me | CF₃ | Na⁺ | |
| 1-94 | Me | Et | SEt | CF₃ | Na⁺ | |
| 1-95 | Me | Et | SOEt | CF₃ | Na⁺ | |
| 1-96 | Me | Et | SO₂Et | CF₃ | Na⁺ | |
| 1-97 | Me | Et | SMe | Cl | Na⁺ | |
| 1-98 | Et | Et | SMe | Cl | Na⁺ | |
| 1-99 | Me | Et | SOMe | Cl | Na⁺ | |
| 1-100 | Me | Et | SEt | Cl | Na⁺ | |
| 1-101 | Me | Et | SOEt | Cl | Na⁺ | |
| 1-102 | Me | Et | SO₂Et | Cl | Na⁺ | |
| 1-103 | Me | Et | SMe | Br | Na⁺ | |
| 1-104 | Me | Et | SO₂Me | Br | Na⁺ | |
| 1-105 | Me | Pr | SMe | CF₃ | Na⁺ | |
| 1-106 | Me | Pr | SOMe | CF₃ | Na⁺ | |
| 1-107 | Me | c-Pr | SMe | CF₃ | Na⁺ | |
| 1-108 | Me | c-Pr | SOMe | CF₃ | Na⁺ | |
| 1-109 | Me | c-Pr | SO₂Me | CF₃ | Na⁺ | |
| 1-110 | Me | CH₂OMe | SMe | CF₃ | Na⁺ | |
| 1-111 | Me | CH₂OMe | SOMe | CF₃ | Na⁺ | |
| 1-112 | Me | CH₂OMe | SO₂Me | CF₃ | Na⁺ | |
| 1-113 | Me | CH₂OMe | SEt | CF₃ | Na⁺ | |
| 1-114 | Me | CH₂OMe | SOEt | CF₃ | Na⁺ | |
| 1-115 | Me | CH₂OMe | SO₂Et | CF₃ | Na⁺ | |
| 1-116 | Me | CH₂OMe | SMe | SO₂Me | Na⁺ | |
| 1-117 | Me | CH₂OMe | SOMe | SO₂Me | Na⁺ | |
| 1-118 | Me | CH₂OMe | SO₂Me | SO₂Me | Na⁺ | |
| 1-119 | Me | OMe | SMe | CF₃ | Na⁺ | |
| 1-120 | Me | OMe | SMe | CF₃ | Me₃N(CH₂CH₂OH)⁺ | |
| 1-121 | Me | OMe | SOMe | CF₃ | Na⁺ | |
| 1-122 | Me | OMe | SOMe | CF₃ | Me₃N(CH₂CH₂OH)⁺ | |
| 1-123 | Me | OMe | SO₂Me | CF₃ | Na⁺ | |
| 1-124 | Me | OMe | SO₂Me | CF₃ | Me₃N(CH₂CH₂OH)⁺ | |
| 1-125 | Me | OMe | SMe | CHF₂ | Na⁺ | |
| 1-126 | Me | OMe | SMe | CHF₂ | Pr₄N⁺ | |
| 1-127 | Me | OMe | SMe | CHF₂ | Me₃N(CH₂CH₂OH)⁺ | |
| 1-128 | Et | OMe | SMe | CHF₂ | Na⁺ | |
| 1-129 | Et | OMe | SMe | CHF₂ | Pr₄N⁺ | |
| 1-130 | Et | OMe | SMe | CHF₂ | Me₃N(CH₂CH₂OH)⁺ | |
| 1-131 | Me | OMe | SOMe | CHF₂ | Na⁺ | |
| 1-132 | Me | OMe | SOMe | CHF₂ | Pr₄N⁺ | |
| 1-133 | Me | OMe | SOMe | CHF₂ | Me₃N(CH₂CH₂OH)⁺ | |
| 1-134 | Et | OMe | SOMe | CHF₂ | Na⁺ | |
| 1-135 | Et | OMe | SOMe | CHF₂ | Pr₄N⁺ | |
| 1-136 | Et | OMe | SOMe | CHF₂ | Me₃N(CH₂CH₂OH)⁺ | |
| 1-137 | Me | OMe | SO₂Me | CHF₂ | Na⁺ | |
| 1-138 | Me | OMe | SO₂Me | CHF₂ | Pr₄N⁺ | |
| 1-139 | Me | OMe | SO₂Me | CHF₂ | Me₃N(CH₂CH₂OH)⁺ | |
| 1-140 | Et | OMe | SO₂Me | CHF₂ | Na⁺ | |
| 1-141 | Et | OMe | SO₂Me | CHF₂ | Pr₄N⁺ | |
| 1-142 | Et | OMe | SO₂Me | CHF₂ | Me₃N(CH₂CH₂OH)⁺ | |
| 1-143 | Me | OMe | SEt | CF₃ | Na⁺ | |
| 1-144 | Me | OMe | SOEt | CF₃ | Na⁺ | |
| 1-145 | Me | OMe | SO₂Et | CF₃ | Na⁺ | |
| 1-146 | Me | Cl | SMe | H | Na⁺ | |
| 1-147 | Me | Cl | SO₂Me | Me | Na⁺ | |
| 1-148 | Me | Cl | SO₂Et | Me | Na⁺ | |
| 1-149 | Me | Cl | SMe | CF₃ | Na⁺ | |
| 1-150 | Me | Cl | SOMe | CF₃ | Na⁺ | 7.80 (d,1H), 7.64 (d,1H), 3.10 (s, 3H), 2.28 (s, 3H) |
| 1-151 | Me | Cl | SOMe | CF₃ | Et₃N(Bn)⁺ | 7.68 (d,1H), 7.53 (d,1H), 7.47-7.41 (m, 5H); 4.65 (s, 2H); 3.35 (q, 6H), 3.09 (s, 3H); 2.38 (s, 3H); 1.43 (t, 9H) |
| 1-152 | Me | Cl | SOMe | Cl | Pr₄N⁺ | 7.75 (d, 1H), 7.63 (d, 1H), 3.27-3.23 (m, 8H); 3.10 (s, 3H); 2.39 (s, 3H); 1.73-1.67 (m, 8H); 0.95 (t, 12H) |
| 1-153 | Me | Cl | SOMe | Cl | Me₃N(CH₂CH₂OH)⁺ | B7.74 (d, 1H), 7.65 (d, 1H), 4.06 (bs, 2H); 3.72 (bs, 2H); 3.28 (s, 9H); 3.11 (s, 3H); 2.38 (s, 3H) |
| 1-154 | Me | Cl | SOMe | Cl | Me₃N(Bn)⁺ | 7.74 (d,1H), 6.65 (d,1H), 7.50-7.45 (m, 5H); 4.73 (s, 2H); 3.26 (s,9H), 3.10 (s,3H); 2.41 (s, 3H) |
| 1-155 | Me | Cl | SOMe | Cl | n-Oct₄N⁺ | 7.75 (d,1H), 7.62 (d,1H), 3.29-3.25 (m, 8H); 3.09 (s,3H); 2.37 (s, 3H); 1.64 (m, 8H); 1.28-1.24 (m, 48H); 0.87 (t, 12H) |
| 1-156 | Me | Cl | SOMe | CF₃ | Et₃N(Bn)⁺ | 7.68 (d,1H), 7.53 (d,1H), 7.47-7.41 (m, 5H); 4.65 (s, 2H); 3.35 (q,6H), 3.09 (s,3H); 2.38 (s, 3H); 1.43 (t, 9H) |
| 1-157 | Me | Cl | SOMe | CF₃ | Li⁺ | 7.84 (d,1H), 7.71 (d,1H), 3.11 (s,3H), 2.33 (s,3H) |
| 1-158 | Me | Cl | SOMe | CF₃ | K⁺ | 7.82 (d,1H), 7.68 (d,1H), 3.10 (s,3H), 2.30 (s,3H) |
| 1-159 | Me | Cl | SOMe | CF₃ | Mg²⁺ | |
| 1-160 | Me | Cl | SOMe | CF₃ | Ca²⁺ | |
| 1-161 | Me | Cl | SOMe | CF₃ | Me₃S⁺ | |
| 1-162 | Me | Cl | SOMe | CF₃ | Et₃S⁺ | |
| 1-163 | Me | Cl | SOMe | CF₃ | Me₄N⁺ | 7.78 (d,1H), 7.61 (d,1H), 3.10 (s, 12H), 3.09 (s,3H), 2.26 (s,3H) |
| 1-164 | Me | Cl | SOMe | CF₃ | Et₄N⁺ | 7.83 (d,1H), 7.69 (d,1H), 3.20 (q, 8H), 3.10 (s,3H), 1.15 (t, 12H) |
| 1-165 | Me | Cl | SOMe | CF₃ | Bu₄N⁺ | |
| 1-166 | Me | Cl | SOMe | CF₃ | i-Pr₄N⁺ | |
| 1-167 | Me | Cl | SO₂Me | CF₃ | Na⁺ | 7.94 (d,1H), 7.76 (d,1H), 3.49 (s,3H), 2.28 (s,3H) |
| 1-168 | Me | Cl | SO₂Me | CF₃ | Me₃N(CH₂CH₂OH)⁺ | |
| 1-169 | Me | Cl | SO₂Me | CF₃ | Et₄N⁺ | 7.91 (d,1H), 7.73 (d,1H), 3.47 (s,3H), 3.19 (q, 8H), 2.28 (s,3H), 1.16 (t, 12H). |
| 1-170 | c-Pr | Cl | SO₂Me | CF₃ | Na⁺ | 7.93 (d,1H), 7.75 (d,1H), 3.50 (s,3H), 2.00 - 1.96 (m, 1H), 0.99-0.94 (m, 2H), 0.84-0.80 (m, 2H) |
| 1-171 | c-Pr | Cl | SO₂Me | CF₃ | Pr₄N⁺ | |
| 1-172 | Me | Cl | SO₂Me | c-Pr | Na⁺ | |
| 1-173 | Me | Cl | SO₂Et | CF₃ | Na⁺ | |
| 1-174 | Me | Cl | SOEt | c-Pr | Na⁺ | 7.41 (d,1H), 6.92 (d,1H), 3.41-3.19 (m, 2H), 2.28 (s, 3H), 1.24 (t, 3H), 1.08-0.98 (m, 3H), 0.64-0.57 (m, 1H) |
| 1-175 | Me | Cl | SO₂Et | c-Pr | Na⁺ | |
| 1-176 | Me | Cl | SCH₂-c-Pr | c-Pr | Na⁺ | |
| 1-177 | Me | Cl | SOCH₂-c-Pr | c-Pr | Na⁺ | |
| 1-178 | Me | Cl | SO₂CH₂-c-Pr | c-Pr | Na⁺ | |
| 1-179 | Me | Cl | S(CH₂)₂OMe | c-Pr | Na⁺ | |
| 1-180 | Me | Cl | SO(CH₂)₂OMe | c-Pr | Na⁺ | |
| 1-181 | Me | Cl | SO₂(CH₂)₂OMe | c-Pr | Na⁺ | |
| 1-182 | Me | Cl | 1H-pyrazol-1-yl | CF₃ | Na⁺ | 7.96 (d,1H), 7.82 (d,1H), 7.75 (d, 1H), 7.74 (d,1H), 6.51 (dd, 1H), 3.32 (s,3H), 2.28 (s, 3H) |
| 1-183 | Me | Cl | 1H-pyrazol-1-yl | CF₃ | Me₃N(CH₂CH₂OH)⁺ | |
| 1-184 | Me | Cl | 1H-pyrazol-1-yl | C₂F₅ | Na⁺ | |
| 1-185 | Me | Cl | 4-CF₃-1H-pyrazol-1-yl | CF₃ | Na⁺ | |
| 1-186 | Me | Cl | 4-Me-1H-pyrazol-1-yl | CF₃ | Na⁺ | |
| 1-187 | Me | Cl | 2H-1,2,3-triazol-2-yl | CF₃ | Na⁺ | |
| 1-188 | Me | Cl | 2H-1,2,3-triazol-2-yl | C₂F₅ | Na⁺ | |
| 1-189 | Me | Cl | 1H-1,2,3-triazol-1-yl | CF3 | Na⁺ | |
| 1-190 | Me | Cl | 1H-1,2,3-triazol-1-yl | C₂F₅ | Na⁺ | |
| 1-191 | Me | Cl | 1H-1,2,4-triazol-1-yl | CF₃ | Na⁺ | |
| 1-192 | Me | Cl | 1H-1,2,4-triazol-1-yl | C₂F₅ | Na⁺ | |
| 1-193 | Me | Cl | 1H-pyrazol-1-yl | SO₂Me | Na⁺ | |
| 1-194 | Me | Cl | 1H-pyrazol-1-yl | SO₂Me | Me₃N(CH₂CH₂OH)⁺ | |
| 1-195 | Me | Cl | 2-Br-1H-pyrazol-1-yl | SO₂Me | Na⁺ | |
| 1-196 | Me | Cl | 4-CF₃-1H-pyrazol-1-yl | SO₂Me | Na⁺ | |
| 1-197 | Me | Cl | 4-Me-1H-pyrazol-1-yl | SO₂Me | Na⁺ | |
| 1-198 | Me | Cl | 2H-1,2,3-triazol-2-yl | SO₂Me | Na⁺ | |
| 1-199 | Me | Cl | 1H-1,2,4-triazol-1-yl | SO₂Me | Na⁺ | |
| 1-200 | Me | Cl | 1H-1,2,3-triazol-1-yl | SO₂Me | Na⁺ | |
| 1-201 | Me | Cl | OCH₂CH₂OMe | Cl | Na⁺ | |
| 1-202 | Me | Cl | SMe | Cl | Na⁺ | |
| 1-203 | Et | Cl | SMe | Cl | Na⁺ | |
| 1-204 | Me | Cl | SOMe | Cl | Na⁺ | |
| 1-205 | Et | Cl | SOMe | Cl | Na⁺ | |
| 1-206 | Me | Cl | SO₂Me | Cl | Na⁺ | |
| 1-207 | Et | Cl | SO₂Me | Cl | Na⁺ | |
| 1-208 | Me | Cl | SEt | Cl | Na⁺ | |
| 1-209 | Me | Cl | SOEt | Cl | Na⁺ | |
| 1-210 | Me | Cl | SO₂Et | Cl | Na⁺ | |
| 1-211 | Me | Cl | SCH₂CH₂OMe | SO₂Me | Na⁺ | |
| 1-212 | Me | Cl | SOCH₂CH₂OMe | SO₂Me | Na⁺ | |
| 1-213 | Me | Cl | SO₂CH₂CH₂OMe | SO₂Me | Na⁺ | |
| 1-214 | Me | Cl | CH₂OMe | SO₂Me | Na⁺ | |
| 1-215 | Me | Cl | CH₂OMe | SO₂Me | K⁺ | |
| 1-216 | Me | Cl | CH₂OMe | SO₂Me | Pr₄N⁺ | |
| 1-217 | Me | Cl | CH₂OMe | SO₂Me | Me₃N(CH₂CH₂OH)⁺ | |
| 1-218 | Me | Cl | CH₂OCH₂CF₃ | SO₂Me | Na⁺ | |
| 1-219 | Et | Cl | CH₂OCH₂CF₃ | SO₂Me | Na⁺ | |
| 1-220 | Me | Cl | CH₂OCH₂CH₂OMe | SO₂Me | Na⁺ | |
| 1-221 | Me | Cl | Ac | SO₂Me | Na⁺ | |
| 1-222 | Me | Cl | (CO)-c-Pr | SO₂Me | Na⁺ | |
| 1-223 | Me | Cl | C(=NOMe)Me | SO₂Me | Na⁺ | |
| 1-224 | Me | Cl | C(=NOEt)Me | SO₂Me | Na⁺ | |
| 1-225 | Me | Cl | 5-c-Pr-isoxazol-3-yl | SO₂Me | Na⁺ | |
| 1-226 | Me | Cl | 5-Methoxymethyl-1,2-oxazol-3-yl | SO₂Me | Na⁺ | |
| 1-227 | Me | Cl | 3-Methyl-4,5-dihydro-1,2-oxazol-5-yl | SO₂Me | Na⁺ | |
| 1-228 | Me | Cl | 4,5-Dihydro-1,2-oxazol-3-yl | SO₂Me | Na⁺ | |
| 1-229 | Me | Cl | 2H-1,2,3-triazol-2-yl | SO₂Me | Na⁺ | |
| 1-230 | Me | Cl | 2H-1,2,3-triazol-2-yl | SO₂Me | Me₃N(CH₂CH₂OH)⁺ | |
| 1-231 | Me | Cl | 4,5-Dihydro-1,2-oxazol-3-yl | SO₂Et | Na⁺ | |
| 1-232 | Me | Cl | 5-Cyanomethy-4,5-dihydro-1,2-oxazol-3-yl | SO₂Et | Na⁺ | |
| 1-233 | Me | Cl | 5-Cyanomethy-4,5-dihydro-1,2-oxazol-3-yl | SO₂Et | Pr₄N⁺ | |
| 1-234 | Me | Cl | 5-Cyanomethy-4,5-dihydro-1,2-oxazol-3-yl | SO₂Et | Me₃N(CH₂CH₂OH)⁺ | |
| 1-235 | Me | Cl | 5-Cyanomethy-4,5-dihydro-1,2-oxazol-3-yl | SO₂Et | Li⁺ | |
| 1-236 | Me | Cl | 5-Cyanomethy-4,5-dihydro-1,2-oxazol-3-yl | SO₂Et | K⁺ | |
| 1-237 | Me | Cl | 5-Cyanomethy-4,5-dihydro-1,2-oxazol-3-yl | SO₂Et | Mg²⁺ | |
| 1-238 | Me | Cl | 5-Cyanomethy-4,5-dihydro-1,2-oxazol-3-yl | SO₂Et | Ca²⁺ | |
| 1-239 | Me | Cl | 5-Cyanomethy-4,5-dihydro-1,2-oxazol-3-yl | SO₂Et | Me₃S⁺ | |
| 1-240 | Me | Cl | 5-Cyanomethy-4,5-dihydro-1,2-oxazol-3-yl | SO₂Et | Et₃S⁺ | |
| 1-241 | Me | Cl | 5-Cyanomethy-4,5-dihydro-1,2-oxazol-3-yl | SO₂Et | Me₄N⁺ | |
| 1-242 | Me | Cl | 5-Cyanomethy-4,5-dihydro-1,2-oxazol-3-yl | SO₂Et | Et₄N⁺ | |
| 1-243 | Et | Cl | 5-Methoxymethy-4,5-dihydro-1,2-oxazol-3-yl | SO₂Et | Na⁺ | 7.88 (d,1H), 7.71 (d,1H), 5.02-4.89 (m, 1H), 3.60-3.31 (m, 9H); 3.11-3.01 (m, 1H); 1.18(t, 3H), 1.09 (t, 3H) |
| 1-244 | Et | Cl | 5-Methoxymethy-4,5-dihydro-1,2-oxazol-3-yl | SO₂Et | Et₄N⁺ | 7.86 (d,1H), 7.67 (d,1H), 5.01-4.89 (m, 1H), 3.59-3.23 (m, 9H); 3.22-3.17 (m, 8H), 3.11-3.01 (m, 1H); 1.20-1.09 (m, 18H) |
| 1-245 | Me | Cl | OMe | SO₂Me | Na⁺ | |
| 1-246 | Me | Cl | OMe | SO₂Et | | |
| 1-247 | Me | Cl | OEt | SO₂Me | Na⁺ | 7.75 (d,1H), 7.40 (d,1H), 4.19 (q, 2H), 3.32 (s,3H), 2.32 (s, 3H), 1.41 (t, 3H) |
| 1-248 | Me | Cl | OEt | SO₂Et | Na⁺ | |
| 1-249 | Me | Cl | OPr | SO₂Me | Na⁺ | |
| 1-250 | Me | Cl | OPr | SO₂Et | Na⁺ | |
| 1-251 | Me | Cl | O-CHF₂ | SO₂Me | Na⁺ | |
| 1-252 | Me | Cl | O-Propargyl | SO₂Me | Na⁺ | |
| 1-253 | Me | Cl | OCH₂c-Pr | SO₂Me | Na⁺ | |
| 1-254 | Me | Cl | OCH₂c-Pr | SO₂Et | Na⁺ | 7.68 (d,1H), 7.35 (d,1H), 3.98-3.91 (m ,2H), 3.51-3.44 (m, 2H), 2.28 (s, 3H), 1.38 - 1.35 (m, 1H), 1.10 (t, 3H), 0.63-0.59 (m, 2H), 0.43-0.39 (m, 2H) |
| 1-255 | Me | Cl | O(CH₂)₂Cl | SO₂Me | Na+ | |
| 1-256 | Me | Cl | O(CH₂)₂F | SO₂Me | Na+ | |
| 1-257 | Me | Cl | OCH₂CF₃ | SO₂Et | Na⁺ | |
| 1-258 | Me | Cl | O(CH₂)₃OMe | SO₂Me | Na+ | 7.70 (d,1H), 7.35 (d,1H), 4.19 (t, 2H), 3.54 (t, 2H), 3.27 (s,3H), 2.28 (s, 3H), 2.10 - 2.05 (m, 2H) |
| 1-259 | Me | Cl | OCH₂-1,3-dioxolan-2-yl | SO₂Me | Na⁺ | |
| 1-260 | Me | Cl | SMe | SO₂Me | Na⁺ | |
| 1-261 | Me | Cl | SOMe | SO₂Me | Na⁺ | |
| 1-262 | Me | Cl | SO₂Me | SO₂Me | Na⁺ | |
| 1-263 | Me | Cl | SEt | SO₂Me | Na⁺ | |
| 1-264 | Me | Cl | SOEt | SO₂Me | Na⁺ | |
| 1-265 | Me | Cl | SO₂Et | SO₂Me | Na⁺ | |
| 1-266 | Me | Cl | SCH₂CH₂OMe | SO₂Me | Na⁺ | |
| 1-267 | Me | Cl | SOCH₂CH₂OMe | SO₂Me | Na⁺ | |
| 1-268 | Me | Cl | SO₂CH₂CH₂OMe | SO₂Me | Na⁺ | |
| 1-269 | Me | Br | 1H-pyrazol-1-yl | CF₃ | Na⁺ | |
| 1-270 | Me | Br | 1H-pyrazol-1-yl | C₂F₅ | Na⁺ | |
| 1-271 | Me | Br | 2H-1,2,3-triazol-2-yl | CF₃ | Na⁺ | |
| 1-272 | Me | Br | 2H-1,2,3-triazol-2-yl | C₂F₅ | Na⁺ | |
| 1-273 | Me | Br | 1H-1,2,3-triazol-1-yl | CF3 | Na⁺ | |
| 1-274 | Me | Br | 1H-1,2,3-triazol-1-yl | C₂F₅ | Na⁺ | |
| 1-275 | Me | Br | 1H-1,2,4-triazol-1-yl | CF₃ | Na⁺ | |
| 1-276 | Me | Br | 1H-1,2,4-triazol-1-yl | C₂F₅ | Na⁺ | |
| 1-277 | Me | Me | SMe | Me | Na+ | |
| 1-278 | Me | Me | SOMe | Me | Na+ | |
| 1-279 | Me | Me | SO₂Me | Me | Na+ | |
| 1-280 | Me | Me | SEt | Me | Na+ | |
| 1-281 | Me | Me | SOEt | Me | Na+ | |
| 1-282 | Me | Me | SO₂Et | Me | Na+ | |
| 1-283 | Me | Me | S-c-Pr | Me | Na+ | |
| 1-284 | Me | Me | SO-c-Pr | Me | Na+ | |
| 1-285 | Me | Me | SO₂-c-Pr | Me | Na+ | |
| 1-286 | Me | Me | SCH₂-c-Pr | Me | Na+ | |
| 1-287 | Me | Me | SOCH₂-c-Pr | Me | Na+ | |
| 1-288 | Me | Me | SO₂CH₂c-Pr | Me | Na+ | |
| 1-289 | Me | Me | SCH₂CH₂OMe | Me | Na+ | |
| 1-290 | Me | Me | SOCH₂CH₂OMe | Me | Na+ | |
| 1-291 | Me | Me | SO₂CH₂CH₂OMe | Me | Na+ | |
| 1-292 | Me | Me | SMe | Et | Na⁺ | |
| 1-293 | Me | Me | SOMe | Et | Na⁺ | |
| 1-294 | Me | Me | SO₂Me | Et | Na⁺ | |
| 1-295 | Me | Me | SEt | Et | Na⁺ | |
| 1-296 | Me | Me | SOEt | Et | Na⁺ | |
| 1-297 | Me | Me | SO₂Et | Et | Na⁺ | |
| 1-298 | Me | Me | S-c-Pr | Et | Na⁺ | |
| 1-299 | Me | Me | SO-c-Pr | Et | Na⁺ | |
| 1-300 | Me | Me | SO₂-c-Pr | Et | Na⁺ | |
| 1-301 | Me | Me | SCH₂-c-Pr | Et | Na+ | |
| 1-302 | Me | Me | SOCH₂-c-Pr | Et | Na+ | |
| 1-303 | Me | Me | SO₂CH₂-c-Pr | Et | Na+ | |
| 1-304 | Me | Me | SCH₂CH₂OMe | Et | Na+ | |
| 1-305 | Me | Me | SOCH₂CH₂OMe | Et | Na+ | |
| 1-306 | Me | Me | SO₂CH₂CH₂OMe | Et | Na+ | |
| 1-307 | Me | Me | SMe | i-Pr | Na+ | |
| 1-308 | Me | Me | SOMe | i-Pr | Na+ | |
| 1-309 | Me | Me | SO₂Me | i-Pr | Na+ | |
| 1-310 | Me | Me | SEt | i-Pr | Na+ | |
| 1-311 | Me | Me | SOEt | i-Pr | Na+ | |
| 1-312 | Me | Me | SO₂Et | i-Pr | Na+ | |
| 1-313 | Me | Me | S-c-Pr | i-Pr | Na+ | |
| 1-314 | Me | Me | SO-c-Pr | i-Pr | Na+ | |
| 1-315 | Me | Me | SO₂-c-Pr | i-Pr | Na+ | |
| 1-316 | Me | Me | SCH₂-c-Pr | i-Pr | Na+ | |
| 1-317 | Me | Me | SOCH₂-c-Pr | i-Pr | Na+ | |
| 1-318 | Me | Me | SO₂CH₂-c-Pr | i-Pr | Na+ | |
| 1-319 | Me | Me | SCH₂CH₂OMe | i-Pr | Na+ | |
| 1-320 | Me | Me | SOCH₂CH₂OMe | i-Pr | Na+ | |
| | Me | Me | SO₂CH₂CH₂OMe | i-Pr | Na+ | |
| 1-321 | Me | | | | | |
| | Me | | | | | |
| | Me | | | | | |
| 1-322 | Me | Et | SMe | Me | Na+ | |
| 1-323 | Me | Et | SOMe | Me | Na+ | |
| 1-324 | Me | Et | SO₂Me | Me | Na+ | |
| 1-325 | Me | Et | SEt | Me | Na+ | |
| 1-326 | Me | Et | SOEt | Me | Na+ | |
| 1-327 | Me | Et | SO₂Et | Me | Na+ | |
| 1-328 | Me | Et | S-c-Pr | Me | Na+ | |
| 1-329 | Me | Et | SO-c-Pr | Me | Na+ | |
| 1-330 | Me | Et | SO₂-c-Pr | Me | Na+ | |
| 1-331 | Me | Et | SCH₂-c-Pr | Me | Na+ | |
| 1-332 | Me | Et | SOCH₂c-Pr | Me | Na+ | |
| 1-333 | Me | Et | SO₂CH₂-c-Pr | Me | Na+ | |
| 1-334 | Me | Et | SCH₂CH₂OMe | Me | Na+ | |
| 1-335 | Me | Et | SOCH₂CH₂OMe | Me | Na+ | |
| 1-336 | Me | Et | SO₂CH₂CH₂OMe | Me | Na+ | |
| 1-337 | Me | Et | SMe | Et | Na+ | |
| 1-338 | Me | Et | SOMe | Et | Na+ | |
| 1-339 | Me | Et | SO₂Me | Et | Na+ | |
| 1-340 | Me | Et | SEt | Et | Na+ | |
| 1-341 | Me | Et | SOEt | Et | Na+ | |
| 1-342 | Me | Et | SO₂Et | Et | Na+ | |
| 1-343 | Me | Et | S-c-Pr | Et | Na+ | |
| 1-344 | Me | Et | SO-c-Pr | Et | Na+ | |
| 1-345 | Me | Et | SO₂-c-Pr | Et | Na+ | |
| 1-346 | Me | Et | SCH₂-c-Pr | Et | Na+ | |
| 1-347 | Me | Et | SOCH₂-c-Pr | Et | Na+ | |
| 1-348 | Me | Et | SO₂CH₂c-Pr | Et | Na+ | |
| 1-349 | Me | Et | SCH₂CH₂OMe | Et | Na+ | |
| 1-350 | Me | Et | SOCH₂CH₂OMe | Et | Na+ | |
| 1-351 | Me | Et | SO₂CH₂CH₂OMe | Et | Na+ | |
| 1-352 | Me | Et | SMe | i-Pr | Na+ | |
| 1-353 | Me | Et | SOMe | i-Pr | Na+ | |
| 1-354 | Me | Et | SO₂Me | i-Pr | Na+ | |
| 1-355 | Me | Et | SEt | i-Pr | Na+ | |
| 1-356 | Me | Et | SOEt | i-Pr | Na+ | |
| 1-357 | Me | Et | SO₂Et | i-Pr | Na+ | |
| 1-358 | Me | Et | S-c-Pr | i-Pr | Na+ | |
| 1-359 | Me | Et | SO-c-Pr | i-Pr | Na+ | |
| 1-360 | Me | Et | SO₂-c-Pr | i-Pr | Na+ | |
| 1-361 | Me | Et | SCH₂-c-Pr | i-Pr | Na+ | |
| 1-362 | Me | Et | SOCH₂-c-Pr | i-Pr | Na+ | |
| 1-363 | Me | Et | SO₂CH₂-c-Pr | i-Pr | Na+ | |
| 1-364 | Me | Et | SCH₂CH₂OMe | i-Pr | Na+ | |
| 1-365 | Me | Et | SOCH₂CH₂OMe | i-Pr | Na+ | |
| 1-366 | Me | Et | SO₂CH₂CH₂OMe | i-Pr | Na+ | |
| 1-367 | Me | c-Pr | SMe | Me | Na+ | |
| 1-368 | Me | c-Pr | SOMe | Me | Na+ | |
| 1-369 | Me | c-Pr | SO₂Me | Me | Na+ | |
| 1-370 | Me | c-Pr | SEt | Me | Na+ | |
| 1-371 | Me | c-Pr | SOEt | Me | Na+ | |
| 1-372 | Me | c-Pr | SO₂Et | Me | Na+ | |
| 1-373 | Me | c-Pr | S-c-Pr | Me | Na+ | |
| 1-374 | Me | c-Pr | SO-c-Pr | Me | Na+ | |
| 1-375 | Me | c-Pr | SO₂-c-Pr | Me | Na+ | |
| 1-376 | Me | c-Pr | SCH₂-c-Pr | Me | Na+ | |
| 1-377 | Me | c-Pr | SOCH₂-c-Pr | Me | Na+ | |
| 1-378 | Me | c-Pr | SO₂CH₂-c-Pr | Me | Na+ | |
| 1-379 | Me | c-Pr | SCH₂CH₂OMe | Me | Na+ | |
| 1-380 | Me | c-Pr | SOCH₂CH₂OMe | Me | Na+ | |
| 1-381 | Me | c-Pr | SO₂CH₂CH₂OMe | Me | Na+ | |
| 1-382 | Me | c-Pr | SMe | Et | Na+ | |
| 1-383 | Me | c-Pr | SOMe | Et | Na+ | |
| 1-384 | Me | c-Pr | SO₂Me | Et | Na+ | |
| 1-385 | Me | c-Pr | SEt | Et | Na+ | |
| 1-386 | Me | c-Pr | SOEt | Et | Na+ | |
| 1-387 | Me | c-Pr | SO₂Et | Et | Na+ | |
| 1-388 | Me | c-Pr | S-c-Pr | Et | Na+ | |
| 1-389 | Me | c-Pr | SO-c-Pr | Et | Na+ | |
| 1-390 | Me | c-Pr | SO₂-c-Pr | Et | Na+ | |
| 1-391 | Me | c-Pr | SCH₂-c-Pr | Et | Na+ | |
| 1-392 | Me | c-Pr | SOCH₂-c-Pr | Et | Na+ | |
| 1-393 | Me | c-Pr | SO₂CH₂-c-Pr | Et | Na+ | |
| 1-394 | Me | c-Pr | SCH₂CH₂OMe | Et | Na+ | |
| 1-395 | Me | c-Pr | SOCH₂CH₂OMe | Et | Na+ | |
| 1-396 | Me | c-Pr | SO₂CH₂CH₂OMe | Et | Na+ | |
| 1-397 | Me | c-Pr | SMe | i-Pr | Na+ | |
| 1-398 | Me | c-Pr | SOMe | i-Pr | Na+ | |
| 1-399 | Me | c-Pr | SO₂Me | i-Pr | Na+ | |
| 1-400 | Me | c-Pr | SEt | i-Pr | Na+ | |
| 1-401 | Me | c-Pr | SOEt | i-Pr | Na+ | |
| 1-402 | Me | c-Pr | SO₂Et | i-Pr | Na+ | |
| 1-403 | Me | c-Pr | S-c-Pr | i-Pr | Na+ | |
| 1-404 | Me | c-Pr | SO-c-Pr | i-Pr | Na+ | |
| 1-405 | Me | c-Pr | SO₂-c-Pr | i-Pr | Na+ | |
| 1-406 | Me | c-Pr | SCH₂-c-Pr | i-Pr | Na+ | |
| 1-407 | Me | c-Pr | SOCH₂-c-Pr | i-Pr | Na+ | |
| 1-408 | Me | c-Pr | SO₂CH₂-c-Pr | i-Pr | Na+ | |
| 1-409 | Me | c-Pr | SCH₂CH₂OMe | i-Pr | Na+ | |
| 1-410 | Me | c-Pr | SOCH₂CH₂OMe | i-Pr | Na+ | |
| 1-411 | Me | c-Pr | SO₂CH₂CH₂OMe | i-Pr | Na+ | |

Die Verbindungen der Nummern 1-19, 1-20, 1-22, 1-23, 1-25, 1-32, 1-34, 1-42 bis 1-52, 1-65 bis 1-77, 1-88 bis 1-90, 1-108 bis 1-118, 1-120, 1-122, 1-124, 1-126, 1-127, 1-129, 1-130, 1-132, 1-133, 1-135, 1-136, 1-138, 1-139, 1-141, 1-142, 1-146, 1-151 bis 1-156, 1-161 bis 1-166, 1-168, 1-169, 1-171, 1-176 bis 1-201, 1-211 bis 1-244, 1-251 bis 1-259, 1-266 bis 1-276, 1-283 bis 1-291, 1-298 bis 1-306, 1-313 bis 1-321, 1-328 bis 1-336, 1-343 bis 1-351, 1-358 bis 1-366, 1-373 bis 1-381, 1-388 bis 1-396 und 1-403 bis 1-411 sind Referenzbeispiele.

**Tabelle 2: Verbindungen der allgemeinen Formel (I), worin A für N steht**

| | | | | | |
|---|---|---|---|---|---|
| 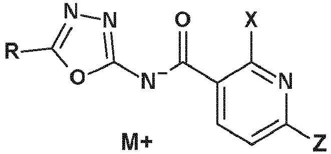 | | | | | |

| **Nr.** | **R** | **X** | **Z** | **M⁺** | **Physikalische Daten** |
|---|---|---|---|---|---|
| | | | | | **(¹H-NMR, DMSO-d₆, 400 MHz)** |
| 2-1 | Me | Me | CF₃ | Na⁺ | 8,16 (d,1H), 7.68 (d,1H), 3.18 (s,3H), 2.33 (s,3H) |
| 2-2 | Me | Me | CF₃ | Et₃N(Bn)⁺ | |
| 2-3 | Me | Me | CF₃ | Pr₄N⁺ | 8,08 (d,1H), 7.62 (d,1H), 3.14-3.10 (m, 8H), 2.68 (s,3H), 2.26 (s,3H), 1.64-1.58 (m, 8H), 0.89 (t, 12H) |
| 2-4 | Me | Me | CF₃ | Me₃N(CH₂CH₂OH)⁺ | 8,08 (d,1H), 7.62 (d,1H), 5.48 (bs, 1H), 3.85-3.82 (m, 2H), 3.41-3.38 (m, 2H), 3.10 (s, 12H), 2.68 (s,3H), 2.27 (s,3H) |
| 2-5 | Me | Me | CF₃ | Me₃N(Bn)⁺ | 8,14 (d,1H), 7.70 (d,1H), 7.55-7.49 (m, 5H), 4.52 (s, 2H), 3.02 (s, 12H), 2.67 (s,3H), 2.34 (s,3H) |
| 2-6 | Me | Me | CF₃ | n-Oct₄N⁺ | 8,08 (d,1H), 7.62 (d,1H), 3.17-3.13 (m, 8H), 2.68 (s,3H), 2.26 (s,3H), 1.62-1.49 (m, 8H), 1.32-1.18 (m, 40H), 0.86 (t, 12H) |
| 2-7 | Me | Me | CF₃ | Et₃N(Bn)⁺ | 8,08 (d,1H), 7.61 (d,1H), 7.53-7.51 (m, 5H), 4.47 (s, 2H(, 3.16 (q, 8H), 2.68 (s,3H), 2.26 (s,3H), 1.30 (t, 12H) |
| 2-8 | Me | Me | CF₃ | K⁺ | 8,13 (d,1H), 7.68 (d,1H), 2.68 (s,3H), 2.33 (s,3H) |
| 2-9 | Me | Me | CF₃ | Li⁺ | 8,26 (d,1H), 7.70 (d,1H), 2.75 (s,3H), 2.34 (s,3H) |
| 2-10 | Me | Me | CF₃ | Mg²⁺ | |
| 2-11 | Me | Me | CF₃ | Ca²⁺ | |
| 2-12 | Me | Me | CF₃ | Me₃S⁺ | |
| 2-13 | Me | Me | CF₃ | Et₃S⁺ | |
| 2-14 | Me | Me | CF₃ | Me₄N⁺ | 8,20 (d,1H), 7.80 (d,1H), 3.10 (s,12H), 2.66 (s,3H), 2.42 (s, 3H) |
| 2-15 | Me | Me | CF₃ | Et₄N⁺ | 8,13 (d,1H), 7.69 (d,1H), 3.20 (q, 8H), 2.67 (s,3H), 2.32 (s, 3H), 1.16 (t, 12H) |
| 2-16 | Me | Me | CF₃ | Bu₄N⁺ | |
| 2-17 | Me | Me | CF₃ | i-Pr₄N⁺ | |
| 2-18 | Me | Cl | CF₃ | Na⁺ | |
| 2-19 | Me | Cl | CF₃ | Pr₄N⁺ | |
| 2-20 | Me | Br | CF₃ | Na⁺ | |
| 2-21 | Me | CH₂OMe | CF₃ | Na⁺ | |

Die Verbindungen der Nummern 2-2 bis 2-7, 2-12 bis 2-17, 2-19 und 2-21 sind Referenzbeispiele.

### B. Formulierungsbeispiele

a) Ein Stäubemittel wird erhalten, indem man 10 Gew.-Teile einer Verbindung der Formel (I) und/oder deren Salze und 90 Gew.-Teile Talkum als Inertstoff mischt und in einer Schlagmühle zerkleinert.
b) Ein in Wasser leicht dispergierbares, benetzbares Pulver wird erhalten, indem man 25 Gewichtsteile einer Verbindung der Formel (I) und/oder deren Salze, 64 Gew.-Teile kaolinhaltigen Quarz als Inertstoff, 10 Gewichtsteile ligninsulfonsaures Kalium und 1 Gew.-Teil oleoylmethyltaurinsaures Natrium als Netz- und Dispergiermittel mischt und in einer Stiftmühle mahlt.
c) Ein in Wasser leicht dispergierbares Dispersionskonzentrat wird erhalten, indem man 20 Gew.-Teile einer Verbindung der Formel (I) und/oder deren Salze mit 6 Gew.-Teilen Alkylphenolpolyglykolether (®Triton X 207), 3 Gew.-Teilen Isotridecanolpolyglykolether (8 EO) und 71 Gew.-Teilen paraffinischem Mineralöl (Siedebereich z.B. ca. 255 bis über 277 C) mischt und in einer Reibkugelmühle auf eine Feinheit von unter 5 Mikron vermahlt.
d) Ein emulgierbares Konzentrat wird erhalten aus 15 Gew.-Teilen einer Verbindung der Formel (I) und/oder deren Salze, 75 Gew.-Teilen Cyclohexanon als Lösungsmittel und 10 Gew.-Teilen oxethyliertes Nonylphenol als Emulgator.
e) Ein in Wasser dispergierbares Granulat wird erhalten indem man
   75 Gew.-Teile einer Verbindung der Formel (I) und/oder deren Salze,
   10 Gew.-Teile ligninsulfonsaures Calcium,
   5 Gew.-Teile Natriumlaurylsulfat,
   3 Gew.-Teile Polyvinylalkohol und
   7 Gew.-Teile Kaolin
   mischt, auf einer Stiftmühle mahlt und das Pulver in einem Wirbelbett durch Aufsprühen von Wasser als Granulierflüssigkeit granuliert.
f) Ein in Wasser dispergierbares Granulat wird auch erhalten, indem man
   25 Gew.-Teile einer Verbindung der Formel (I) und/oder deren Salze,
   5 Gew.-Teile 2,2'-dinaphthylmethan-6,6'-disulfonsaures Natrium
   2 Gew.-Teile oleoylmethyltaurinsaures Natrium,
   1 Gew.-Teil Polyvinylalkohol,
   17 Gew.-Teile Calciumcarbonat und
   50 Gew.-Teile Wasser
   auf einer Kolloidmühle homogenisiert und vorzerkleinert, anschließend auf einer Perlmühle mahlt und die so erhaltene Suspension in einem Sprühturm mittels einer Einstoffdüse zerstäubt und trocknet.

### C. Biologische Beispiele

### 1. Herbizide Wirkung gegen Schadpflanzen im Vorauflauf

Samen von mono- bzw. dikotylen Schad- bzw. Kulturpflanzen werden in Holzfasertöpfen in sandiger Lehmerde ausgelegt und mit Erde abgedeckt. Die in Form von benetzbaren Pulvern (WP) oder als Emulsionskonzentrate (EC) formulierten erfindungsgemäßen Verbindungen sowie zu Vergleichszwecken die aus WO 2012/126932 A1 bekannten strukturell ähnlichsten Verbindungen werden dann als wäßrige Suspension bzw. Emulsion mit einer Wasseraufwandmenge von umgerechnet 600 bis 800 l/ha unter Zusatz von 0,2% Netzmittel auf die Oberfläche der Abdeckerde appliziert. Nach der Behandlung werden die Töpfe im Gewächshaus aufgestellt und unter guten Wachstumsbedingungen für die Testpflanzen gehalten. Die visuelle Bonitur der Schäden an den Versuchspflanzen erfolgt nach einer Versuchszeit von 3 Wochen im Vergleich zu unbehandelten Kontrollen (herbizide Wirkung in Prozent (%): 100% Wirkung = Pflanzen sind abgestorben, 0 % Wirkung = wie Kontrollpflanzen). Dabei zeigen die getesteten erfindungsgemäßen Verbindungen eine bessere Wirkung gegen Schadpflanzen und gleichzeitig eine bessere Verträglichkeit, d.h. geringere Schädigung gegenüber Kulturpflanzen. Die Vergleichsversuche wurden beispielhaft an einigen Schad- bzw. Kulturpflanzen durchgeführt.

Die hier verwendeten Abkürzungen bedeuten:

| | | | |
|---|---|---|---|
| ABUTH | Abutilon theophrasti | AMARE | Amaranthus retroflexus |
| POLCO | Polygonum convolvulus | STEME | Stellaria media |
| TRZAS | Triticum aestivum (Weizen) | ZEAMX | Zea mays (Mais) |

**Tabelle V1**

| **Verbindung Nr.** | **Dosierung [g/ha]** | **Wirkung gegen ABUTH** | ***Schädigung von TRZAS*** |
|---|---|---|---|
| 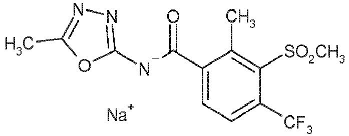 | 20 | 100% | *0%* |
| Nr. 1-14, erfindungsgemäß | | | |
| 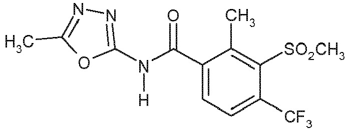 | 20 | 70% | *40%* |
| Nr. 2-145, aus WO 2012/126932 | | | |

**Tabelle V2**

| **Verbindung Nr.** | **Dosierung [g/ha]** | **Wirkung gegen STEME** | ***Schädigung von TRZAS*** |
|---|---|---|---|
| 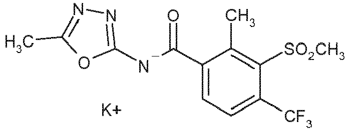 | 20 | 90% | 0% |
| Nr. 1-16, erfindungsgemäß | | | |
| 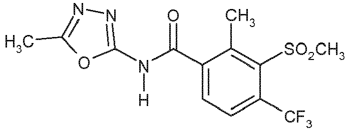 | 20 | 70% | *40%* |
| Nr. 2-145, aus WO 2012/126932 | | | |

**Tabelle V3**

| **Verbindung Nr.** | **Dosierung [g/ha]** | **Wirkung gegen STEME** | ***Schädigung von TRZAS*** |
|---|---|---|---|
| 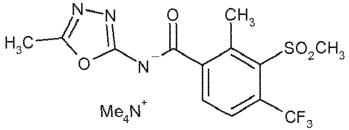 | 20 | 90% | *0%* |
| Nr. 1-21, erfindungsgemäß | | | |
| 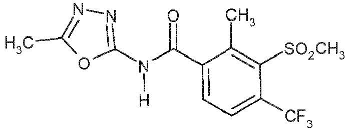 | 20 | 70% | *40%* |
| Nr. 2-145, aus WO 2012/126932 | | | |

**Tabelle V4**

| **Verbindung Nr.** | **Dosierung [g/ha]** | **Wirkung gegen ABUTH** | ***Schädigung von TRZAS*** |
|---|---|---|---|
| 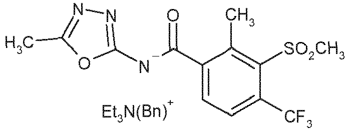 | 20 | 100% | *0%* |
| Nr. 1-25, erfindungsgemäß | | | |
| 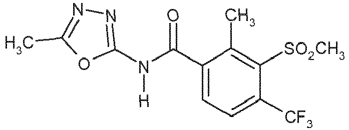 | 20 | 70% | *40%* |
| Nr. 2-145, aus WO 2012/126932 | | | |

**Tabelle V5**

| **Verbindung Nr.** | **Dosierung [g/ha]** | **Wirkung gegen ABUTH** | ***Schädigung von TRZAS*** |
|---|---|---|---|
| 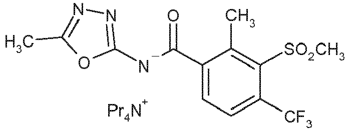 | 20 | 100% | *0%* |
| Nr. 1-26, erfindungsgemäß | | | |
| 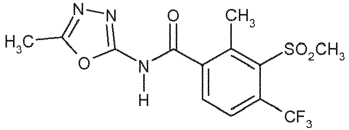 | 20 | 70% | *40%* |
| Nr. 2-145, aus WO 2012/126932 | | | |

**Tabelle V6**

| **Verbindung Nr.** | **Dosierung [g/ha]** | **Wirkung gegen ABUTH** | ***Schädigung von TRZAS*** |
|---|---|---|---|
| 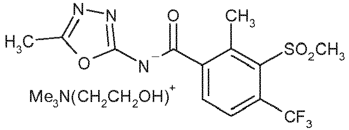 | 20 | 100% | *0%* |
| Nr. 1-27, erfindungsgemäß | | | |
| 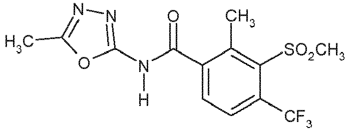 | 20 | 70% | *40%* |
| Nr. 2-145, aus WO 2012/126932 | | | |

**Tabelle V7**

| **Verbindung Nr.** | **Dosierung [g/ha]** | **Wirkung gegen ABUTH** | ***Schädigung von TRZAS*** |
|---|---|---|---|
| 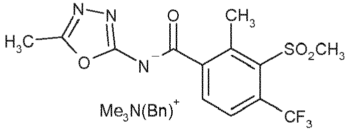 | 20 | 100% | *0%* |
| Nr. 1-28, erfindungsgemäß | | | |
| 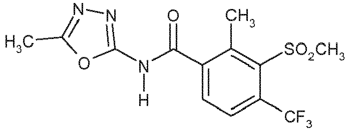 | 20 | 70% | *40%* |
| Nr. 2-145, aus WO 2012/126932 | | | |

| Tabelle V8 | | | |
|---|---|---|---|
| **Verbindung Nr.** | **Dosierung [g/ha]** | **Wirkung gegen ABUTH** | ***Schädigung von TRZAS*** |
| 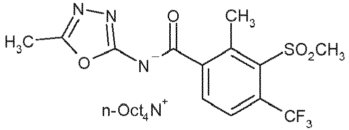 | 20 | 100% | *0%* |
| Nr. 1-29, erfindungsgemäß | | | |
| 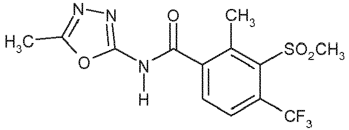 | 20 | 70% | *40%* |
| Nr. 2-145, aus WO 2012/126932 | | | |

**Tabelle V9**

| **Verbindung Nr.** | **Dosierun g [g/ha]** | **Wirkung gegen** | | | ***Schädigun g von ZEAMX*** |
|---|---|---|---|---|---|
| | | **ALOMY** | **CYPES** | **ABUTH** | |
| 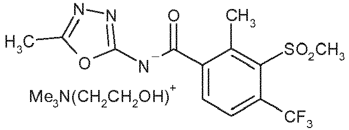 | 20 | 70% | 70% | 100% | *0%* |
| Nr. 1-27, erfindungsgemäß | | | | | |
| 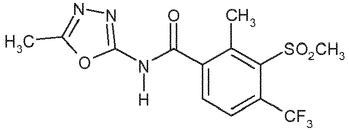 | 20 | 20% | 40% | 70% | *0%* |
| Nr. 2-145, aus WO 2012/126932 | | | | | |

**Tabelle V10**

| **Verbindung Nr.** | **Dosierun g [g/ha]** | **Wirkung gegen** | | | ***Schädigun g von ZEAMX*** |
|---|---|---|---|---|---|
| | | **ALOMY** | **CYPES** | **ABUTH** | |
| 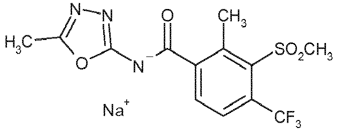 | 20 | 60% | 40% | 100% | *0%* |
| Nr. 1-14, erfindungsgemäß | | | | | |
| 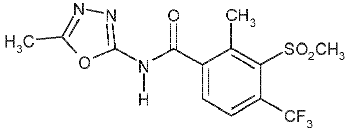 | 20 | 20% | 40% | 70% | 0% |
| Nr. 2-145, aus WO 2012/126932 | | | | | |

**Tabelle V11**

| **Verbindung Nr.** | **Dosierun g [g/ha]** | **Wirkung gegen** | | | ***Schädigun g von ZEAMX*** |
|---|---|---|---|---|---|
| | | **ALOMY** | **CYPES** | **ABUTH** | |
| 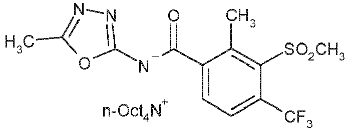 | 20 | 60% | 60% | 100% | *0%* |
| Nr. 1-29, erfindungsgemäß | | | | | |
| 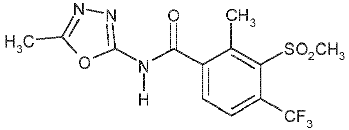 | 20 | 20% | 40% | 70% | *0%* |

**Tabelle V12**

| **Verbindung Nr.** | **Dosierun g [g/ha]** | **Wirkung gegen** | | | ***Schädigun g von ZEAMX*** |
|---|---|---|---|---|---|
| | | **ALOMY** | **CYPES** | **ABUTH** | |
| 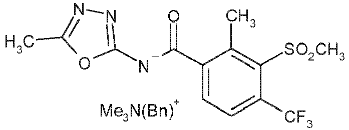 | 20 | 10% | 70% | 100% | *10%* |
| Nr. 1-28, erfindungsgemäß | | | | | |
| 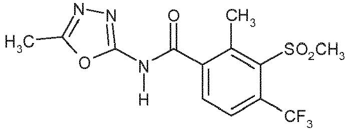 | 20 | 20% | 40% | 70% | *0%* |
| Nr. 2-145, aus WO 2012/126932 | | | | | |

**Tabelle V13**

| **Verbindung Nr.** | **Dosierung [g/ha]** | **Wirkung gegen** | | | ***Schädigung von ZEAMX*** |
|---|---|---|---|---|---|
| | | **ALOMY** | **CYPES** | **ABUTH** | |
| 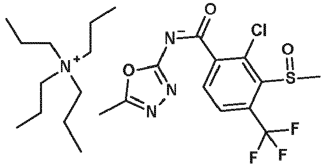 | 20 | 0% | 80% | 90% | *0%* |
| Nr. 1-152, | | | | | |
| 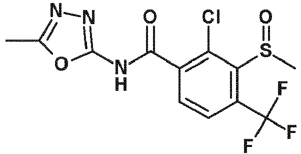 | 20 | 20% | 60% | 60% | *0%* |
| Nr. 2-360, aus WO 2012/126932 | | | | | |

**Tabelle V14**

| **Verbindung Nr.** | **Dosierung [g/ha]** | **Wirkung gegen** | | | ***Schädigung von ZEAMX*** |
|---|---|---|---|---|---|
| | | **ALOMY** | **CYPES** | **ABUTH** | |
| 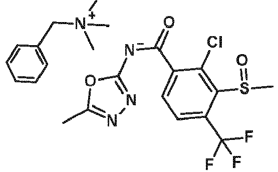 | 20 | 60% | 80% | 90% | *0%* |
| Nr. 1-154, | | | | | |
| 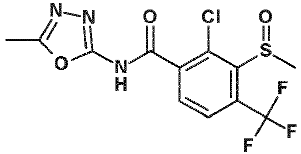 | 20 | 20% | 60% | 60% | *0%* |
| Nr. 2-360, aus WO 2012/126932 | | | | | |

**Tabelle V15**

| **Verbindung Nr.** | **Dosierung [g/ha]** | **Wirkung gegen** | | | ***Schädigung von ZEAMX*** |
|---|---|---|---|---|---|
| | | **ALOMY** | **CYPES** | **ABUTH** | |
| 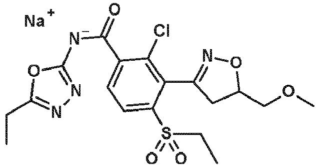 | 320 | 30% | 80% | 90% | *0%* |
| Nr. 1-243, | | | | | |
| 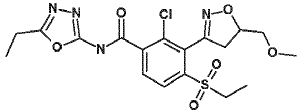 | 320 | 10% | 80% | 90% | *0%* |
| Nr. 3-265, aus WO 2012/126932 | | | | | |

**Tabelle V16**

| **Verbindung Nr.** | **Dosierung [g/ha]** | **Wirkung gegen** | | | ***Schädigung von ZEAMX*** |
|---|---|---|---|---|---|
| | | **ALOMY** | **CYPES** | **ABUTH** | |
| 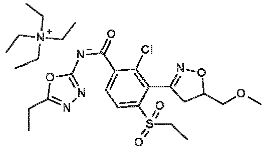 | 320 | 20% | 100% | 80% | *10%* |
| Nr. 1-244, | | | | | |
| 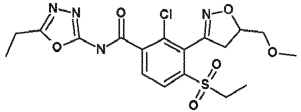 | 320 | 10% | 80 | 10% | *0%* |
| Nr. 3-265, aus WO 2012/126932 | | | | | |

### 2. Herbizide Wirkung gegen Schadpflanzen im Nachauflauf

Samen von mono- bzw. dikotylen Unkraut- bzw. Kulturpflanzen werden in Holzfasertöpfen in sandigem Lehmboden ausgelegt, mit Erde abgedeckt und im Gewächshaus unter guten Wachstumsbedingungen angezogen. 2 bis 3 Wochen nach der Aussaat werden die Versuchspflanzen im Einblattstadium behandelt. Die in Form von benetzbaren Pulvern (WP) oder als Emulsionskonzentrate (EC) formulierten erfindungsgemäßen Verbindungen sowie zu Vergleichszwecken die aus WO 2012/126932 A1 bekannten strukturell ähnlichsten Verbindungen werden dann als wäßrige Suspension bzw. Emulsion mit einer Wasseraufwandmenge von umgerechnet 600 bis 800 l/ha unter Zusatz von 0,2% Netzmittel auf die grünen Pflanzenteile gesprüht. Nach ca. 3 Wochen Standzeit der Versuchspflanzen im Gewächshaus unter optimalen Wachstumsbedingungen wird die Wirkung der Präparate visuell im Vergleich zu unbehandelten Kontrollen bonitiert (herbizide Wirkung in Prozent (%): 100% Wirkung = Pflanzen sind abgestorben, 0 % Wirkung = wie Kontrollpflanzen). Dabei zeigen die getesteten erfindungsgemäßen Verbindungen eine bessere Wirkung gegen Schadpflanzen und gleichzeitig eine bessere Verträglichkeit, d.h. geringere Schädigung gegenüber Kulturpflanzen. Die Vergleichsversuche wurden beispielhaft an einigen Schad- bzw. Kulturpflanzen durchgeführt.

**Tabelle N1**

| **Verbindung Nr.** | **Dosierung [g/ha]** | **Wirkung gegen AMARE** | ***Schädigung von ZEAMX*** |
|---|---|---|---|
| 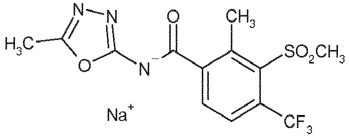 | 5 | 100% | *0%* |
| Nr. 1-14, erfindungsgemäß | | | |
| 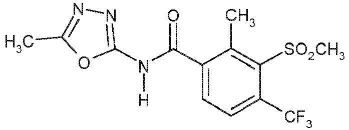 | 5 | 80% | *40%* |
| Nr. 2-145, aus WO 2012/126932 | | | |

**Tabelle N2**

| **Verbindung Nr.** | **Dosierung [g/ha]** | **Wirkung gegen AMARE** | ***Schädigung von ZEAMX*** |
|---|---|---|---|
| 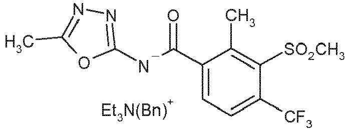 | 5 | 100% | *0%* |
| Nr. 1-25, erfindungsgemäß | | | |
| 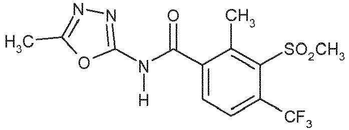 | 5 | 80% | *40%* |
| Nr. 2-145, aus WO 2012/126932 | | | |

**Tabelle N3**

| **Verbindung Nr.** | **Dosierung [g/ha]** | **Wirkung gegen AMARE** | ***Schädigung von ZEAMX*** |
|---|---|---|---|
| 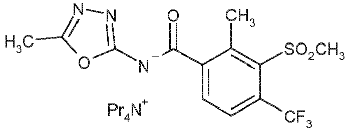 | 5 | 100% | *0%* |
| Nr. 1-26, erfindungsgemäß | | | |
| 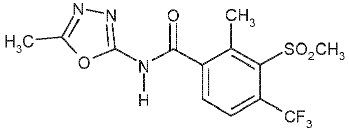 | 5 | 80% | *40%* |
| Nr. 2-145, aus WO 2012/126932 | | | |

**Tabelle N4**

| **Verbindung Nr.** | **Dosierung [g/ha]** | **Wirkung gegen AMARE** | ***Schädigung von ZEAMX*** |
|---|---|---|---|
| 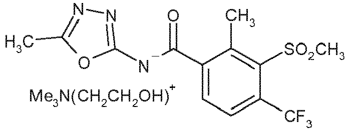 | 5 | 100% | *0%* |
| Nr. 1-27, erfindungsgemäß | | | |
| 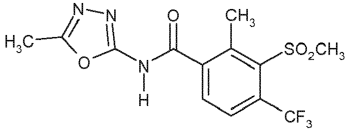 | 5 | 80% | *40%* |
| Nr. 2-145, aus WO 2012/126932 | | | |

**Tabelle N5**

| **Verbindung Nr.** | **Dosierung [g/ha]** | **Wirkung gegen POLCO** | ***Schädigung von ZEAMX*** |
|---|---|---|---|
| 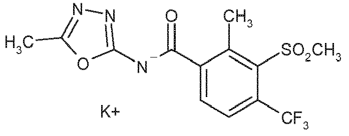 | 5 | 60% | *0%* |
| Nr. 1-16, erfindungsgemäß | | | |
| 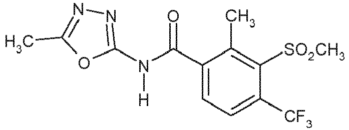 | 5 | 40% | *40%* |
| Nr. 2-145, aus WO 2012/126932 | | | |

**Tabelle N6**

| **Verbindung Nr.** | **Dosierung [g/ha]** | **Wirkung gegen POLCO** | ***Schädigung von ZEAMX*** |
|---|---|---|---|
| 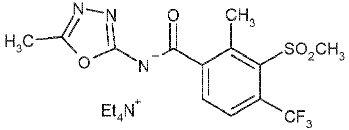 | 5 | 60% | *0%* |
| Nr. 1-22, erfindungsgemäß | | | |
| 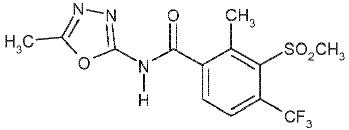 | 5 | 40% | *40%* |
| Nr. 2-145, aus WO 2012/126932 | | | |

**Tabelle N7**

| **Verbindung Nr.** | **Dosierung [g/ha]** | **Wirkung gegen** | | ***Schädigung von TRZAS*** |
|---|---|---|---|---|
| | | **ALOMY** | **VERPE** | |
| 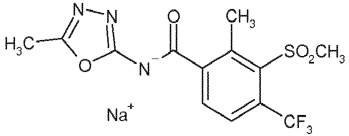 | 5 | 80% | 100% | *0%* |
| Nr. 1-14, erfindungsgemäß | | | | |
| 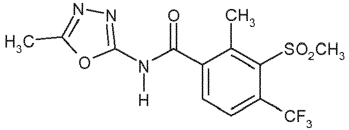 | 5 | 60 | 60% | *80%* |
| Nr. 2-145, aus WO 2012/126932 | | | | |

**Tabelle N8**

| **Verbindung Nr.** | **Dosierung [g/ha]** | **Wirkung gegen** | | ***Schädigung von TRZAS*** |
|---|---|---|---|---|
| | | **ALOMY** | **VERPE** | |
| 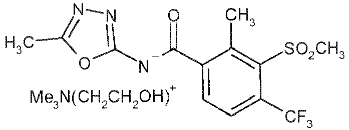 | 5 | 80% | 90% | *0%* |
| Nr. 1-27, erfindungsgemäß | | | | |
| 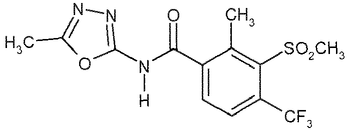 | 5 | 60% | 60% | *80%* |
| Nr. 2-145, aus WO 2012/126932 | | | | |

**Tabelle N9**

| **Verbindung Nr.** | **Dosierung [g/ha]** | **Wirkung gegen** | | ***Schädigung von TRZAS*** |
|---|---|---|---|---|
| | | **ALOMY** | **VERPE** | |
| 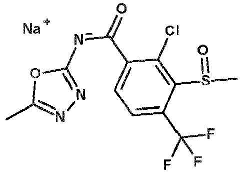 | 5 | 80% | 70% | *0%* |
| Nr.1-150, erfindungsgemäß | | | | |
| 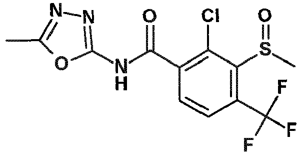 | 5 | 60% | 50% | *30%* |
| Nr. 2-360, aus WO 2012/126932 | | | | |

**Tabelle N10**

| **Verbindung Nr.** | **Dosierung [g/ha]** | **Wirkung gegen** | | ***Schädigung von TRZAS*** |
|---|---|---|---|---|
| | | **ALOMY** | **VERPE** | |
| 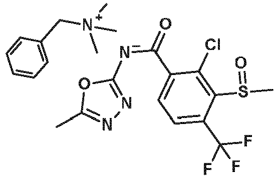 | 5 | 80% | 80% | *0%* |
| Nr. 1-154, | | | | |
| 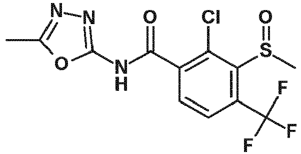 | 5 | 60% | 50% | *30%* |
| Nr. 2-360, aus WO 2012/126932 | | | | |

## Patentansprüche

1. Salze von N-(1,3,4-Oxadiazol-2-yl)benzamiden der Formel (I) 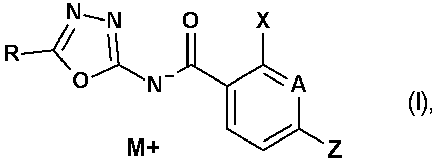 worin
A bedeutet N oder CY,
R bedeutet (C₁-C₆)-Alkyl, (C₃-C₇)-Cycloalkyl oder Methoxymethyl,
X bedeutet Halogen, (C₁-C₆)-Alkyl, Halogen-(C₁-C₆)-alkyl, OR¹ oder S(O)ₙR²,
Y (C₁-C₆)-Alkyl, (C₁-C₆)-Halogenalkyl, OR¹ oder S(O)ₙR²,
Z bedeutet Halogen, Methyl, Halogen-(C₁-C₆)-alkyl oder S(O)ₙR²,
R¹ bedeutet Wasserstoff, (C₁-C₆)-Alkyl oder (C₃-C₆)-Cycloalkyl,
R² bedeutet (C₁-C₆)-Alkyl,
n bedeutet 0, 1 oder 2,
M+ bedeutet ein Kation ausgewählt aus der Gruppe bestehend aus Natrium-Ion, Kalium-Ion, Lithium-Ion, Magnesium-Ion und Calcium-Ion.

2. Salze von N-(1,3,4-Oxadiazol-2-yl)benzamiden der Formel (I) 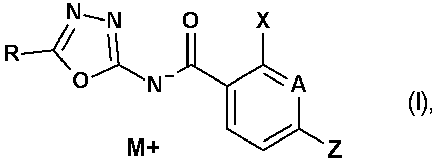 worin
A bedeutet CY,
R bedeutet Methyl,
X bedeutet Methyl,
Y Methylsulfonyl,
Z Trifluormethyl,
M+ bedeutet ein Kation ausgewählt aus der Gruppe bestehend aus Natrium-Ion, Kalium-Ion, N-(2-Hydroxyeth-1-yl)-tris-N,N,N-methylammonium-lon, Tetramethylammonium-Ion, Tetrapropylammonium-Ion, Tetraoctylammonium-Ion und Trimethylbenzylammonium-Ion.

3. Herbizide Mittel, **gekennzeichnet durch** einen herbizid wirksamen Gehalt an mindestens einer Verbindung der Formel (I) gemäß Anspruch 1 oder 2.

4. Herbizide Mittel nach Anspruch 3 in Mischung mit Formulierungshilfsmitteln.

5. Herbizide Mittel nach Anspruch 3 oder 4 enthaltend mindestens einen weiteren pestizid wirksamen Stoff aus der Gruppe Insektizide, Akarizide, Herbizide, Fungizide, Safener und Wachstumsregulatoren.

6. Herbizide Mittel nach Anspruch 5 enthaltend einen Safener.

7. Herbizide Mittel nach Anspruch 6 enthaltend cyprosulfamid, cloquintocet-mexyl, mefenpyr-diethyl oder isoxadifen-ethyl.

8. Herbizide Mittel nach einem der Ansprüche 5 bis 7 enthaltend ein weiteres Herbizid.

9. Verfahren zur Bekämpfung unerwünschter Pflanzen, **dadurch gekennzeichnet, daß** man eine wirksame Menge mindestens einer Verbindung der Formel (I) gemäß Anspruch 1 oder 2 oder eines herbiziden Mittels nach einem der Ansprüche 3 bis 8 auf die Pflanzen oder auf den Ort des unerwünschten Pflanzenwachstums appliziert.

10. Verwendung von Verbindungen der Formel (I) gemäß Anspruch 1 oder 2 oder von herbiziden Mitteln nach einem der Ansprüche 3 bis 8 zur Bekämpfung unerwünschter Pflanzen.

11. Verwendung nach Anspruch 10, **dadurch gekennzeichnet, daß** die Verbindungen der Formel (I) zur Bekämpfung unerwünschter Pflanzen in Kulturen von Nutzpflanzen eingesetzt werden.

12. Verwendung nach Anspruch 11, **dadurch gekennzeichnet, daß** die Nutzpflanzen transgene Nutzpflanzen sind.

## Claims

1. Salts of N-(1,3,4-oxadiazol-2-yl)benzamides of the formula (I) 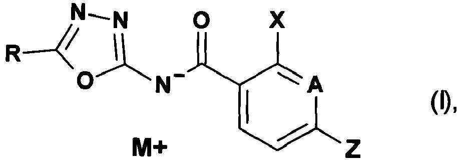 in which
A is N or CY,
R is (C₁-C₆) -alkyl, (C₃-C₇) -cycloalkyl, methoxymethyl,
X is halogen, (C₁-C₆) -alkyl, halo- (C₁-C₆) -alkyl, OR¹, or S(O)ₙR²,
Y is (C₁-C₆) -alkyl, (C₁-C₆) -haloalkyl, OR¹, or S(O)ₙR²,
Z is halogen, methyl, halo- (C₁-C₆) -alkyl, S(O)ₙR²,
R¹ is hydrogen, (C₁-C₆) -alkyl, (C₃-C₆) -cycloalkyl,
R² is (C₁-C₆) -alkyl,
n is 0, 1 or 2,
M+ is a cation selected from the group consisting of sodium ion, potassium ion, lithium ion, magnesium ion, and calcium ion.

2. Salts of N-(1,3,4-oxadiazol-2-yl)benzamides of the formula (I) 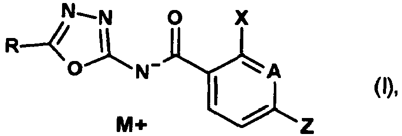 in which
A is CY,
R is methyl,
X is methyl,
Y is methylsulfonyl,
Z is trifluoromethyl,
M+ is a cation selected from the group consisting of sodium ion, potassium ion, N-(2-hydroxyeth-1-yl)-tris-N,N,N-methylammonium ion, tetramethylammonium ion, tetrapropylammonium ion, tetraoctylammonium ion, and trimethylbenzylammonium ion.

3. Herbicidal compositions **characterized by** a herbicidally active content of at least one compound of the formula (I) according to Claim 1 or 2.

4. Herbicidal compositions according to Claim 3 in a mixture with formulation auxiliaries.

5. Herbicidal compositions according to Claim 3 or 4, comprising at least one further pesticidally active substance from the group consisting of insecticides, acaricides, herbicides, fungicides, safeners, and growth regulators.

6. Herbicidal compositions according to Claim 5, comprising a safener.

7. Herbicidal compositions according to Claim 6, comprising cyprosulfamide, cloquintocet-mexyl, mefenpyr-diethyl or isoxadifen-ethyl.

8. Herbicidal compositions according to any of Claims 5 to 7, comprising a further herbicide.

9. Method of controlling unwanted plants, **characterized in that** an effective amount of at least one compound of the formula (I) according to claim 1 or 2 or of a herbicidal composition according to any of Claims 3 to 8 is applied to the plants or to the site of the unwanted vegetation.

10. Use of compounds of the formula (I) according to Claim 1 or 2 or of herbicidal compositions according to any of Claims 3 to 8 for controlling unwanted plants.

11. Use according to Claim 10, **characterized in that** the compounds of the formula (I) are used for controlling unwanted plants in crops of useful plants.

12. Use according to Claim 11, **characterized in that** the useful plants are transgenic useful plants.

## Revendications

1. Sels de N-(1,3,4-oxadiazol-2-yl)benzamides de la formule (I) : 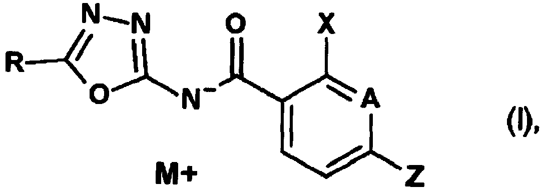 dans laquelle
A signifie N ou CY,
R signifie alkyle en (C₁-C₆), cycloalkyle en (C₃-C₇) ou méthoxyméthyle,
X signifie halogène, alkyle en (C₁-C₆), halogénoalkyle en (C₁-C₆), OR¹ ou S(O)ₙR²,
Y signifie alkyle en (C₁-C₆), halogénoalkyle en (C₁-C₆), OR¹ ou S(O)ₙR²,
Z signifie halogène, méthyle, halogéno-alkyle en (C₁-C₆) ou S(O)ₙR²,
R¹ signifie hydrogène, alkyle en (C₁-C₆) ou cycloalkyle en (C₃-C₆),
R² signifie alkyle en (C₁-C₆),
n signifie 0, 1 ou 2,
M⁺ signifie un cation choisi dans le groupe constitué par un ion sodium, un ion potassium, un ion lithium, un ion magnésium et un ion calcium.

2. Sels de N-(1,3,4-oxadiazol-2-yl)benzamides de la formule (I) : 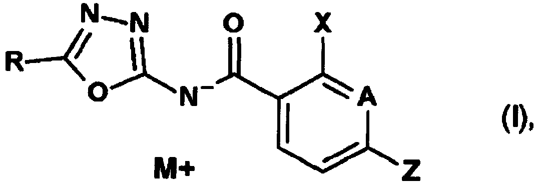 dans laquelle
A signifie CY,
R signifie méthyle,
X signifie méthyle,
Y signifie méthylsulfonyle,
Z signifie trifluorométhyle,
M⁺ signifie un cation choisi dans le groupe constitué par un ion sodium, un ion potassium, un ion N-(2-hydroxyéth-1-yl)-tris-N,N,N-méthylammonium, un ion tétraméthylammonium, un ion tétrapropylammonium, un ion tétraoctylammonium et un ion triméthylbenzylammonium.

3. Agents herbicides, **caractérisés par** une teneur efficace du point de vue herbicide en au moins un composé de la formule (I) selon la revendication 1 ou 2.

4. Agents herbicides selon la revendication 3, en mélange avec des adjuvants de formulation.

5. Agents herbicides selon la revendication 3 ou 4, contenant au moins une substance à activité pesticide supplémentaire du groupe constitué par les insecticides, les acaricides, les herbicides, les fongicides, les agents protecteurs et les régulateurs de croissance.

6. Agents herbicides selon la revendication 5, contenant un agent protecteur.

7. Agents herbicides selon la revendication 6, contenant du cyprosulfamide, du cloquintocet-mexyl, du méfenpyr-diéthyle ou de l'isoxadifène-éthyle.

8. Agents herbicides selon l'une quelconque des revendications 5 à 7, contenant un herbicide supplémentaire.

9. Procédé de lutte contre des plantes indésirables, **caractérisé en ce qu'**une quantité efficace d'au moins un composé de la formule (I) selon la revendication 1 ou 2 ou d'un agent herbicide selon l'une quelconque des revendications 3 à 8 est appliquée sur les plantes ou à l'emplacement de la végétation indésirable.

10. Utilisation de composés de la formule (I) selon la revendication 1 ou 2 ou d'agents herbicides selon l'une quelconque des revendications 3 à 8 pour lutter contre des plantes indésirables.

11. Utilisation selon la revendication 10, **caractérisée en ce que** les composés de la formule (I) sont utilisés pour lutter contre des plantes indésirables dans des cultures de plantes utiles.

12. Utilisation selon la revendication 11, **caractérisée en ce que** les plantes utiles sont des plantes utiles transgéniques.
